# EUROPÄISCHE PATENTSCHRIFT

(11) **EP 1 963 248 B1**
(45) Veröffentlichungstag und Bekanntmachung des Hinweises auf die Patenterteilung: **08.06.2016**
(21) Anmeldenummer: 06830501.0
(22) Anmeldetag: 11.12.2006
(51) Int. Cl.: C07C 57/05

(54) **VERFAHREN DER HETEROGEN KATALYSIERTEN PARTIELLEN GASPHASENOXIDATION VON PROPYLEN ZU ACRYLSÄURE**
METHOD FOR THE HETEROGENEOUSLY CATALYSED PARTIAL GAS PHASE OXIDATION OF PROPYLENE TO FORM ACRYLIC ACID
PROCEDE D'OXYDATION EN PHASE GAZEUSE HETEROGENE ET PARTIELLEMENT CATALYSEE DE PROPYLENE EN ACIDE ACRYLIQUE

(30) Priorität: 22.12.2005 DE 102005062026; 22.12.2005 US 752369 P
(43) Veröffentlichungstag der Anmeldung: 03.09.2008
(73) Patentinhaber: BASF SE, 67056 Ludwigshafen am Rhein (DE)
(72) Erfinder: DIETERLE, Martin, 67063 Ludwigshafen (DE); DIEFENBACHER, Armin, 67361 Freisbach (DE); MÜLLER-ENGEL, Klaus, Joachim, 76297 Stutensee (DE); PETZOLDT, Jochen, 67273 Weisenheim Am Berg (DE)
(86) Internationale Anmeldenummer: PCT/EP2006/069527
(87) Internationale Veröffentlichungsnummer: WO 2007/074045

(56) Entgegenhaltungen:
- WO-A-2004/031106
- DE-A1- 10 245 585

## Beschreibung

Vorliegende Erfindung betrifft ein Verfahren der heterogen katalysierten partiellen Gasphasenoxidation von Propylen zu Acrylsäure, bei dem man in einer ersten Reaktionszone ein Propylen und molekularen Sauerstoff als Reaktanden sowie wenigsten Propan als inertes Verdünnungsgas enthaltendes Reaktionsgasausgangsgemisch 1, das den molekularen Sauerstoff und das Propylen in einem molaren Verhältnis O₂: C₃H₆ ≥ 1 enthält, zunächst in einer ersten Reaktionsstufe bei erhöhter Temperatur so durch wenigstens ein erstes Katalysatorbett führt, dessen Katalysatoren als Aktivmasse wenigstens ein Mo, Fe und Bi enthaltendes Multimetalloxid aufweisen, dass der Propylenumsatz bei einmaligem Durchgang durch das Katalysatorbett ≥ 90 mol-% und die damit einhergehende Selektivität S^{AC} der Acroleinbildung sowie der Acryfsäurenebenproduktbildung zusammen ≥ 80 mol-% betragen, die Temperatur des die erste Reaktionsstufe verlassenden Produktgasgemischs 1 durch direkte Kühlung, oder durch indirekte Kühlung, oder durch direkte und indirekte Kühlung gegebenenfalls verringert, und dem Produktgasgemisch 1 gegebenenfalls Sekundärgas in Form von molekularem Sauerstoff, oder Inertgas, oder molekularem Sauerstoff und Inertgas zugibt, und danach das Produktgasgemisch 1 als Acrolein und molekularen Sauerstoff als Reaktanden sowie wenigstens Propan als inertes Verdünnungsgas enthaltendes Reaktionsgasausgangsgemisch 2, das den molekularen Sauerstoff und das Acrolein in einem molaren Verhältnis O₂: C₃H₄O ≥ 0,5 enthält, in einer zweiten Reaktionsstufe bei erhöhter Temperatur und unter Bildung eines Produktgasgemischs 2 so durch wenigstens ein zweites Katalysatorbett führt, dessen Katalysatoren als Aktivmasse wenigstens ein Mo und V enthaltendes Multimetalloxid aufweisen, dass der Acroleinumsatz bei einmaligem Durchgang durch das Katalysatorbett ≥ 95 mol-% und die Selektivität S^{AA} der über beide Reaktionsstufen bilanzierten Acrylsäurebildung, bezogen auf umgesetztes Propylen, ≥ 70 mol-% beträgt.

Acrylsäure ist als ein Partialoxidationsprodukt des Propylen ein bedeutendes Monomeres, das als solches oder in Form seiner Alkylester zur Erzeugung von z. B. als Klebstoffe geeigneten oder Wasser superabsorbierenden Polymerisaten Verwendung findet (vgl. z. B. WO 03/011804, DE-A 102 45 585, EP-A 1 611 078, DE-A 10 2005 013 039, DE-A 10 2005 010 111, WO 02/055469 und WO 03/078378).

Die Herstellung von Acrylsäure durch heterogen katalysierte zweistufige Partialoxidation von Propylen ist bekannt (vgl. z. B. DE-A 102 45 585, WO 03/011804, DE-A 101 31 297, WO 01/96270).

Das für diese Verfahrensweise als Ausgangssubstanz benötigte Propylen wird als Bestandteil von rohem Propylen (auch "Roh-Propylen") zugesetzt. Im Unterschied zu chemisch reinem Propylen soll in dieser Schrift unter Roh-Propylen Propylen verstanden werden, das neben Propylen noch wenigstens zwei (oder wenigstens drei, oder wenigstens vier) weitere, von Propan und Cyclopropan verschiedene (sowie vorzugsweise auch von Wasser und molekularem Sauerstoff verschiedene) Bestandteile (Verunreinigungen) enthält. Als solche Verunreinigungen kommen, je nach Herstellweg des Roh-Propylen, beispielsweise in Betracht (vgl. z. B. DE-A 101 31 297): Ethan, Methan, C₄-Kohlenwasserstoffe, Acethylen, Ethylen, Wasser, O₂, Schwefel enthaltende Verbindungen, Chlor enthaltende Verbindungen, CO₂, CO, Propadien, Propin, C_{≥5}-Kohlenwasserstoffe, Carbonylgruppen enthaltende Verbindungen u.s.w.. Beispielsweise kann Roh-Propylen auch das Produktgasgemisch einer heterogen katalysierten partiellen Propandehydrierung sein (vgl. z. B. DE-A 102 45 582 und DE-A 10 2005 022 798). Im besonderen soll Roh-Propan in dieser Schrift zusätzlich so beschaffen sein, dass das in ihm enthaltene Propylen zu wenigstens 90 mol-% noch keine heterogen katalysierte Partialoxidation zu Acrylsäure durchlaufen hat. Erfindungsgemäß bevorzugt soll Roh-Propylen zusätzlich zum Vorgenannten nicht aus einer heterogen katalysierten partiellen Dehydrierung von Propan entstammen (bzw. nicht auf eine solche partielle Dehydrierung rückführbar sein). Erfindungsgemäß ganz besonders bevorzugt soll Roh-Propylen zusätzlich zum Vorgenannten weder aus einer heterogen katalysierten Dehydrierung von Propan noch aus einer heterogen katalysierten partiellen Oxidehydrierung von Propan entstammen (bzw. nicht auf solche partiellen Dehydrierungen rückführbar sein). Erfindungsgemäß enthält Roh-Propylen zu wenigstens 90 Gew.-% Propylen. Bevorzugt betragen vorgenannten Propylengehalte des Roh-Propylen erfindungsgemäß vorteilhaft wenigstens 92 Gew.-%, oder wenigstens 94 Gew.-%, oder wenigstens 95 Gew.-%, oder wenigstens 96 Gew.-%, vorteilhaft wenigstens 97 Gew.-%, bevorzugt ≥ 98 Gew.-% und besonders bevorzugt ≥ 99 (oder ≥ 99,5)Gew.-%.

Desweiteren ist es für das erfindungsgemäße Verfahren vorteilhaft, wenn das zu verwendende Roh-Propylen zu ≥ 90 Gew.-% aus Propylen und zu ≥ 98 Gew.-% (vorzugsweise zu ≥ 99 Gew.-%) aus Propan und Propylen besteht.

Günstig ist es für das erfindungsgemäße Verfahren weiterhin, wenn das zu verwendende Roh-Propylen zu ≥ 94 Gew.-% aus Propylen und zu ≥ 97 Gew.-% (bzw. zu ≥ 98 Gew.-%, vorzugsweise zu ≥ 99 Gew.-%) aus Propan und Propylen besteht.

Besonders günstig ist es für das erfindungsgemäße Verfahren ferner, wenn das zu verwendende Roh-Propylen zu ≥ 96 Gew.-% (besser zu ≥ 97 Gew.-%) aus Propylen und zu ≥ 98 Gew.-% (bzw. zu ≥ 99 Gew.-%) aus Propan und Propylen besteht.

Ganz besonders günstig ist es für das erfindungsgemäße Verfahren, wenn das zu verwendende Roh-Propylen zu ≥ 99,6 Gew.-% aus Propylen und zu ≥ 99,7 Gew.-% aus Propan und Propylen besteht.

Grundsätzlich ist es möglich, alle in Roh-Propylen enthaltenen Verunreinigungen vom darin enthaltenen Propylen abzutrennen (vgl. z. B. DE-A 35 21 458 und DE-A 102 45 585). Dies ist jedoch dann nicht erforderlich, wenn sich die Verunreinigungen im Rahmen der heterogen katalysierten Partialoxidation des Propylen zu Acrylsäure inert verhalten. Ist letztere Eigenschaft gegeben, wirken die Verunreinigungen im Reaktionsgasausgangsgemisch während der heterogen katalysierten Partialoxidation von Propylen zu Acrylsäure in einfacher Weise als inerte Verdünnungsgase (vgl. WO 01/96270 und DE-A 33 13 573).

Darunter werden in dieser Schrift ganz allgemein solche Gase verstanden, die im Verlauf der Partialoxidation, jeweils für sich, zu wenigstens 95 mol-%, bevorzugt zu wenigstens 97 mol-% und ganz besonders bevorzugt zu 99 mol-% oder mehr chemisch unverändert erhalten bleiben.

Im Rahmen einer Abtrennung der Acrylsäure vom Produktgasgemisch der Partialoxidation (üblicherweise erfolgt diese durch Überführen der Acrylsäure aus dem Produktgasgemisch in die kondensierte Phase) verbleiben diese inerten Gase üblicherweise als Restgas in der Gasphase und können so nach der Partialoxidation in vergleichsweise einfacher Weise vom Zielprodukt Acrylsäure abgetrennt werden, als dies im Rahmen einer Abtrennung vom Propylen vorab der Partialoxidation der Fall wäre. Das Vorgenannte trifft in entsprechender Weise auf in sonstigen inerten Verdünnungsgasen enthaltene Verunreinigungen zu, falls diese inerten Verdünnungsgase dem Reaktionsgasausgangsgemisch für die Partialoxidation als solche rohen inerten Verdünnungsgase zugesetzt werden.

Als solche Inertgase wurden bisher in der Fachliteratur im Bezug auf die Partialoxidation von Propylen zu Acrylsäure die Propane angesehen. Die Überlegungen gehen diesbezüglich sogar soweit, Propylen als Rohstoff zur Herstellung von Acrylsäure durch Propan als solchen Rohstoff zu ersetzen. In diesem Fall wird Propan in einem ersten Schritt partiell zu Propylen dehydriert und nachfolgend das im ersten Schritt gebildete Propylen im Beisein des nicht umgesetzten Propan heterogen katalysiert zu Acrylsäure partialoxidiert. Normalerweise bildet Propan in einem solchermaßen resultierenden Reaktionsgasausgangsgemisch sogar den Hauptbestandteil. Durch Rückführung des bei der Kondensation des Zielproduktes aus dem Produktgasgemisch verbleibenden, des nicht umgesetztes Propan enthaltenden Restgases in die Dehydrierung und/oder Partialoxidation kann das Propan solchermaßen schließlich vollständig in Acrylsäure überführt werden (vgl. z. B. DE-A 102 45 585, DE-A 10 2005 009 885, DE-A 10 2005 010 111). Zwar kann eine verschwindende Menge des Propan (auf seine Einsatzmenge bezogen größenordnungsmäßig 0,01 Gew.-%) zu Propionsäure (sie ist bereits alleine aufgrund ihres auch in kleinsten Mengen unangenehmen Geruchs sowie wegen ihres radikalischen Polymerisationsunvermögens ein unerwünschter Acrylsäurebegleiter) umgesetzt werden, doch lässt sich einer solchermaßen geringen Nebenproduktbildung z. B. dadurch begegnen, dass man das Reaktionsgasausgangsgemisch 1 zusätzlich mit einem von Propan verschiedenen inerten Verdünnungsgas (z. B. N₂, H₂O, CO₂, Edelgas, Gemische dieser Gase etc.) verdünnt (vgl. z. B. WO 01/96270).

Die vorbeschriebenen Überlegungen sind jedoch dann nicht mehr zutreffend, wenn sich die Roh-Gas-Verunreinigung in einer heterogen katalysierten partiellen Oxidation von Propylen zu Acrylsäure nicht inert verhält, sondern in nennenswerten Anteilen zu einem Nebenprodukt der Acrylsäurebildung umgesetzt wird. Dies ist darauf zurückzuführen, dass das gebildete Nebenprodukt normalerweise nicht als Zielproduktverunreinigung mit selbigem ausgelassen werden kann. Vielmehr wirken in vielen Fällen auch geringfügige Zielproduktverunreinigungen mit Blick auf die angestrebte Zielproduktverwendung als störend (z. B. bei einer Verwendung der Acrylsäure zur Herstellung von Polyacrylsäuren und/oder deren Teil- und/oder vollneutralisierten Alkalisalzen, die überwiegend als Wasser superabsorbierende Materialien im Hygienebereich zum Einsatz kommen; oder bei einer Verwendung der Acrylsäure zur Herstellung ihrer Alkylester und der Verwendung der letzteren zur Herstellung von als Klebstoffen geeigneten Polymerisaten) und müssen dann vergleichsweise aufwändig mittels thermischer Trennverfahren vom Zielprodukt (bzw. der dieses enthaltenden kondensierten Phase) abgetrennt werden (bzw. umgekehrt). In solchen Fällen wird man dann zweckmäßigerweise versuchen, z. B. die entsprechende Propylenverunreinigung vorab der Partialoxidation abzutrennen. Dies vor allem dann, wenn die Roh-Gas-Verunreinigung im Verlauf der heterogen katalysierten Partialoxidation zu Acrylsäure zu einem Nebenprodukt umgesetzt wird, das der Acrylsäure vergleichsweise ähnlich ist.

In vielen Fällen wird aus Gründen der Wirtschaftlichkeit auch parallel vorgegangen. D.h., eine Teilmenge der Roh-Gas-Verunreinigung wird vorab der Roh-Gas Verwendung für die Partialoxidation abgetrennt, und die verbliebene Teilmenge wird nach durchgeführter Partialoxidation als gebildetes Nebenprodukt vom Zielprodukt abgetrennt (bzw. umgekehrt). Ist die verbliebene Teilmenge gering genug, kann das daraus gebildete Nebenprodukt gegebenenfalls auch mit dem Zielprodukt ausgelassen werden. Als mögliche Abtrennverfahren kommen insbesondere thermische Trennverfahren in Betracht.

Unter thermischen Trennverfahren werden dabei solche Verfahren verstanden, bei denen wenigstens zwei voneinander verschiedene stoffliche Phasen (z. B. flüssig/flüssig; gasförmig/flüssig; fest/flüssig; gasförmig/fest etc.) erzeugt und miteinander in Kontakt gebracht werden. Aufgrund des zwischen den Phasen bestehenden Ungleichgewichts findet zwischen denselben ein Wärme- und Stoffaustausch statt, der letztlich die gewünschte Auftrennung (Abtrennung) bedingt. Die Bezeichnung thermische Trennverfahren reflektiert dabei, dass es entweder des Entzuges oder der Zufuhr von Wärme bedarf, um die Ausbildung der stofflichen Phasen zu erzeugen und/oder dass der Entzug oder die Zufuhr von thermischer Energie den Stoffaustausch begünstigt bzw. aufrechterhält.

Thermische Trennverfahren sind daher z. B. Destillationen, Rektifikationen, Kristallisationen, Extraktionen, azeotrope Destillationen, azeotrope Rektifikationen, die Strippung, die Desorption etc. (vgl. auch WO 04/063138). Kristallisative thermische Trennverfahren gelten darunter als besonders investitionsintensiv.

In überraschender Weise wurde nun im Rahmen eigener Arbeiten gefunden, dass Cyclopropan, ein nicht seltener Begleiter von Propylen in Roh-Propylen und von Propan in Roh-Propan, im Rahmen einer wie eingangs beschriebenen heterogen katalysierten Partialoxidation von Propylen zu Acrylsäure im Unterschied zu n-Propan (ohne weiteren Zusatz meint Propan in dieser Schrift stets n-Propan) kein Inertgas bildet. Beim Erhitzen auf 100 bis 200 °C in Gegenwart von Katalysatoren (z. B. Pt) isomerisiert sich Cyclopropan zwar zu Propylen (z. B. Lehrbuch der Oranischen Chemie, Beyer•Walter, Hirzel Verlag Stuttgart, Seite 390, 1991). Im Verlauf einer wie eingangs beschriebenen heterogen katalysierten Partialoxidation von Propylen zu Acrylsäure verhält es sich jedoch gänzlich anders als Propylen und reagiert nicht wie letzteres nahezu ausschließlich zu Acrylsäure, sondern in völlig unerwarteter Weise in hohem und in überraschender Weise erheblichem Umfang zu Propionsäure.

Diese ist im Rahmen thermischer Trennverfahren aber besonders schwierig von Acrylsäure abtrennbar (Sdp. bei 1 bar von Acrylsäure: 141 °C; von Propionsäure: 141,35 °C). Allenfalls kristallisativ sind angemessene Abreicherungskoeffizienten erreichbar. Die Aufgabe der vorliegenden Erfindung bestand daher darin, mit dem vorbeschriebenen überraschenden Zufallsbefund im Kontext mit einer Herstellung von z. B. an Propionsäure möglichst armer Acrylsäure über den Weg einer zweistufigen heterogen katalysierten Partialoxidation von Propylen zu Acrylsäure in vorteilhafter Weise umzugehen. Dies auch vor dem Hintergrund, dass vorgenannte Isomerisierung zu Propylen eine einfache Möglichkeit ist, das Cyclopropan in Roh-Gasen vorab ihrer Verwendung für die Partialoxidation zu beseitigen. Prinzipiell kann Cyclopropan von Propylen bzw. Propan aber auch rektifikativ abgetrennt werden, sind die relevanten Siedepunkte bei Normaldruck doch ausreichend voneinander verschieden (Sdp. Propylen = -47 °C; Sdp. Propan = -44,5 °C und Sdp. Cyclopropan = -32,8 °C). Vorgenannte Aufgabenstellung und ihre Lösung ist vor allem dann von besonderem Interesse, wenn wenigstens eine Teilmenge des bei der Abtrennung der Acrylsäure aus dem Produktgasgemisch der Partialoxidation verbleibenden Restgases, das in der Partialoxidation nicht vollständig umgesetztes Cyclopropan enthalten würde, wenigstens teilweise als Kreisgas in die Partialoxidation als Bestandteil des entsprechenden Reaktionsgasausgangsgemischs rückgeführt wird, ginge mit einer solchen Kreisgasführung im kontinuierlichen Betrieb doch eine Aufpegelung des Cyclopropan im Reaktionsgasausgangsgemisch einher.

Als Lösung der erfindungsgemäßen Aufgabe wurde ein Verfahren der heterogen katalysierten partiellen Gasphasenoxidation von Propylen zu Acrylsäure, bei dem man in einer ersten Reaktionszone ein Propylen und molekularen Sauerstoff als Reaktanden sowie wenigstens Propan als inertes Verdünnungsgas enthaltendes Reaktionsgasausgangsgemisch 1, das den molekularen Sauerstoff und das Propylen in einem molaren Verhältnis O₂: C₃H₆ ≥ 1 enthält, zunächst in einer ersten Reaktionsstufe bei erhöhter Temperatur so durch wenigstens ein Katalysatorbett führt, dessen Katalysatoren als Aktivmasse wenigstens ein Mo, Fe und Bi enthaltendes Multimetalloxid aufweisen, dass der Propylenumsatz U^{P} bei einmaligem Durchgang durch das Katalysatorbett ≥ 90 mol-% und die damit einhergehende Selektivität S^{AC} der Acroleinbildung sowie der Acrylsäurenebenproduktbildung zusammen ≥ 80 (vorzugsweise ≥ 85, bzw. ≥ 90) mol-% betragen, die Temperatur des die erste Reaktionsstufe verlassenden Produktgasgemischs 1 durch direkte Kühlung, oder durch indirekte Kühlung, oder durch direkte und indirekte Kühlung gegebenenfalls verringert, und dem Produktgasgemisch 1 gegebenenfalls Sekundärgas in Form von molekularem Sauerstoff, oder Inertgas, oder molekularem Sauerstoff und Inertgas zugibt, und danach das Produktgasgemisch 1 als Acrolein und molekularen Sauerstoff als Reaktanden sowie wenigstens Propan als inertes Verdünnungsgas enthaltendes Reaktionsgasausgangsgemisch 2, das den molekularen Sauerstoff und das Acrolein in einem molaren Verhältnis O₂: C₃H₄O ≥ 0,5 enthält, in einer zweiten Reaktionsstufe bei erhöhter Temperatur und unter Bildung eines Produktgasgemischs 2 so durch wenigstens ein zweites Katalysatorbett führt, dessen Katalysatoren als Aktivmasse wenigstens ein Mo und V enthaltendes Multime-talloxid aufweisen, dass der Acroleinumsatz U^{A} bei einmaligem Durchgang durch das Katalysatorbett ≥ 95 mol-% und die Selektivität S^{AA} der über beide Reaktionsstufen bilanzierten Acrylsäurebildung, bezogen auf umgesetztes Propylen, ≥ 70 (vorzugsweise ≥ 75 bzw. ≥ 80) mol-% beträgt, gefunden, das dadurch gekennzeichnet ist, dass das Reaktionsgasausgangsgemisch 1, bezogen auf die darin enthaltene molare Menge an Propan, ≤ 3 mol-% Cyclopropan enthält und das für das Verfahren als Ausgangssubstanz benötigte Propylen als Bestandteil von Roh-Propylen zugesetzt wird, das zu mindestens 90 Gew.-% aus Propylen und zu mindestens 97 Gew.-% aus Propan und Propylen besteht.

Das erfindungsgemäße Verfahren ist insbesondere dann von Bedeutung, wenn es so betrieben wird, dass U^{A} ≥ 96 mol-%, bzw. ≥ 97 mol-%, oder ≥ 98 mol-%, oder ≥ 98,5 mol-%, oder ≥ 99 mol-%, bzw. ≥ 99,5 mol-%, oder ≥ 99,8 mol-% oder mehr beträgt. Das Vorgenannte trifft insbesondere dann zu, wenn die erste Reaktionsstufe gleichzeitig so betrieben wird, dass U^{P} ≥ 91 mol-%, bzw. ≥ 92 mol-%, oder ≥ 93 mol-%, oder ≥ 94 mol-%, bzw. ≥ 95 mol-%, oder ≥ 96 mol-%, oder ≥ 97 mol-%, bzw. ≥ 98 mol-%, oder ≥ 99 mol-% beträgt. Dies ist dadurch bedingt, dass vorgenannte Umsätze (sie beziehen sich stets auf einmaligen Durchgang des Reaktionsgasgemischs durch das Katalysatorbett) bei gleichbleibendem Katalysatorsystem normalerweise dann erzielt werden, wenn die Reaktionstemperatur in derjeweiligen Reaktionsstufe erhöht gewählt wird. Erhöhte Temperaturen sind aber insbesondere auch förderlich für die Umsetzung von Cyclopropan zu Propionsäure.

Das erfindungsgemäße Verfahren entfaltet seine Vorteilhaftigkeit insbesondere dann, wenn der Gehalt des Reaktionsgasausgangsgemischs 1 an Cyclopropan, bezogen auf darin enthaltenes Propan, ≤ 2,5 mol-%, besser ≤ 2 mol-%, vorzugsweise ≤ 1,5 mol-%, besser ≤ 1 mol-%, besonders bevorzugt ≤ 0,75 mol-%, besser ≤ 0,5 mol-%, noch besser ≤ 0,25 oder ≤ 0,2 mol-%, mit weiterem Vorteil ≤ 0,17, oder ≤ 0,15, oder ≤ 0,1 mol-% beträgt. Am Besten ist der Gehalt des Reaktionsgasausgangsgemischs 1 an Cyclopropan verschwindend. Aus Gründen der Zweckmäßigkeit wird man ihn jedoch häufig so bemessen, dass er, bezogen auf im Reaktionsgasausgangsgemisch enthaltenes Propan ≥ 10 molppb, oder ≥ 50 molppb, oder ≥ 100 molppb, oder ≥ 1 molppm, oder ≥ 10 molppm beträgt.

Erfindungsgemäße Verfahren sind damit auch solche, bei denen der Cyclopropangehalt des Reaktionsgasausgangsgemischs 1, bezogen auf darin enthaltenes Propan, 10 molppm bis 8000 molppm, oder 10 molppm bis 5000 molppm, oder 100 molppm bis 3000 molppm, oder 200 molppm bis 2500 molppm, oder 300 molppm bis 2000 molppm, oder 400 molppm, bzw. 500 molppm bis 1500 molppm, oder 750 molppm bis 1250 molppm beträgt.

Im übrigen kann das erfindungsgemäße Verfahren wie die an sich bekannten Verfahren der zweistufigen heterogen katalysierten partiellen Gasphasenoxidation von Propylen zu Acrylsäure durchgeführt werden (vgl. z. B. WO 01/36364).

Beispielsweise kann es sich bei den Katalysatorbetten um Festbetten oder um Wirbelbetten handeln. Erfindungsgemäß bevorzugt ist in beiden Reaktionsstufen die Verwendung von Katalysatorfestbetten.

Unter der Belastung des Katalysator(fest)betts mit Reaktionsgasausgangsgemisch wird in dieser Schrift die Menge an Reaktionsgasausgangsgemisch in Normlitern (= NI; das Volumen in Litern, das die entsprechende Menge Reaktionsgasausgangsgemisch bei Normalbedingungen, d.h., bei 25°C und 1 bar, einnehmen würde) verstanden, die pro Stunde durch einen Liter Katalysator(fest)bett geführt wird. Die Belastung des Katalysator(fest)betts kann auch nur auf eine Komponente des Reaktionsgasausgangsgemischs bezogen sein. Dann ist sie die Menge dieser Komponente in Normlitern, die als Bestandteil des entsprechenden Reaktionsgasausgangsgemischs pro Stunde durch einen Liter des Katalysator(fest)betts geführt wird.

Im einzelnen kann die Realisierung der erfindungsgemäß durchzuführenden zweistufigen heterogen katalysierten Partialoxidation von Propylen zu Acrylsäure unter Verwendung eines erfindungsgemäßen Reaktionsgasausgangsgemischs 1 z. B. wie in den Schriften EP-A 700 714 (erste Reaktionsstufe; wie dort beschrieben, aber auch in entsprechender Gegenstromfahrweise von Salzbad und Reaktionsgasausgangsgemisch über den Rohrbündelreaktor), EP-A 700 893 (zweite Reaktionsstufe; wie dort beschrieben, aber auch in entsprechender Gegenstromfahrweise), WO 04/085369 (insbesondere diese Schrift wird als integraler Bestandteil dieser Schrift betrachtet) (als zweistufiges Verfahren), WO 04/085363, DE-A 103 13 212 (erste Reaktionsstufe), EP-A 1 159 248 (als zweistufiges Verfahren), EP-A 1 159 246 (zweite Reaktionsstufe), EP-A 1 159 247 (als zweistufiges Verfahren), DE-A 199 48 248 (als zweistufiges Verfahren), DE-A 101 01 695 (zweistufig), WO 04/085368 (als zweistufiges Verfahren), DE-A 10 2004 021 764 (zweistufig), WO 04/085362 (erste Reaktionsstufe), WO 04/085370 (zweite Reaktionsstufe), WO 04/085365 (zweite Reaktionsstufe), WO 04/085367 (zweistufig), EP-A 990 636, EP-A 1 007 007 und EP-A 1 106 598 beschrieben durchgeführt werden.

Dies gilt insbesondere für alle in diesen Schriften enthaltenen Ausführungsbeispiele. Sie können wie in diesen Schriften beschrieben durchgeführt werden, jedoch mit dem Unterschied, dass als Reaktionsgasausgangsgemisch für die erste Reaktionsstufe ein erfindungsgemäßes Reaktionsgasausgangsgemisch 1 eingesetzt wird. Die übrigen Parameter betreffend kann wie in den Ausführungsbeispielen der genannten Schriften verfahren werden (insbesondere die Katalysatorfestbetten und die Reaktandenbelastung der Katalysatorfestbetten betreffend). Erfolgt beim erfindungsgemäßen Verfahren zwischen den beiden Reaktionsstufen eine Zufuhr von molekularem Sekundärsauerstoff, so erfolgt diese erfindungsgemäß vorzugsweise in Form von Luft. Sie kann aber auch als reiner molekularer Sauerstoff oder auch als ein sonstiges Gemisch aus molekularem Sauerstoff und aus Inertgas erfolgen. Erfindungsgemäß vorteilhaft erfolgt die Sekundärsauerstoffzufuhr in solcher Menge, dass das Produktgasgemisch 2 noch nicht umgesetzten molekularen Sauerstoff enthält. Allerdings kann die für das Gesamtverfahren benötigte Menge an molekularem Sauerstoff auch bereits dem Reaktionsgasausgangsgemisch 1 zugefügt werden. In der Regel wird das molare Verhältnis von im Reaktionsgasausgangsgemisch 1 enthaltenem molekularem Sauerstoff zu in diesem Gemisch enthaltenem Propylen ≥ 1 und ≤ 3 betragen.

Für die jeweilige der beiden Reaktionsstufen geeignete, die erfindungsgemäß geforderten Elemente enthaltende Multimetalloxidkatalysatoren sind vielfach vorbeschrieben und dem Fachmann wohlbekannt. Beispielsweise verweist die EP-A 253 409 auf Seite 5 auf entsprechende US-Patente. Geeignete Katalysatoren für die jeweilige Oxidationsstufe (Reaktionsstufe) offenbaren auch die DE-A 44 31 957, die DE-A 10 2004 025 445 und die DE-A 44 31 949. Dies gilt auch für jene der allgemeinen Formel I in den beiden vorgenannten älteren Schriften. Für die jeweilige Oxidationsstufe (Reaktionsstufe) verwendbare Katalysatoren offenbaren auch die Schriften DE-A 103 25 488, DE-A 103 25 487, DE-A 103 53 954, DE-A 103 44 149, DE-A 103 51 269, DE-A 103 50 812 und DE-A 103 50 822.

Für das erfindungsgemäße Verfahren in der ersten Reaktionsstufe mögliche, Mo, Fe und Bi enthaltende Multimetalloxidaktivmassen sind auch die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 7 00 714 genannten Multimetalloxidaktivmassen.

Ferner eignen sich für die erste Reaktionsstufe des erfindungsgemäßen Verfahrens die Mo, Bi und Fe enthaltenden Multimetalloxidkatalysatoren, die in den Schriften Research Disclosure Nr. 497012 vom 29.08.2005, DE-A 100 46 957, DE-A 100 63 162, DE-C 3 338 380, DE-A 199 02 562, EP-A 15 565, DE-C 2 380 765, EP-A 8 074 65, EP-A 27 93 74, DE-A 330 00 44, EP-A 575897, US-A 4438217, DE-A 19855913, WO 98/24746, DE-A 197 46 210 (diejenigen der allgemeinen Formel II), JP-A 91/294239, EP-A 293 224 und EP-A 700 714 offenbart werden. Dies gilt insbesondere für die beispielhaften Ausführungsformen in diesen Schriften, unter denen jene der EP-A 15565, der EP-A 575897, der DE-A 197 46 210 und der DE-A 198 55 913 besonders bevorzugt werden. Besonders hervorzuheben sind in diesem Zusammenhang ein Katalysator gemäß Beispiel 1 c aus der EP-A 15 565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Oₓ•10SiO₂ aufweist. Ferner sind hervorzuheben das Beispiel mit der laufenden Nr. 3 aus der DE-A 198 55 913 (Stöchiometrie: Mo₁₂Co₇Fe₃Bi_{0,6}K_{0,08}Si_{1,6}Oₓ) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl derjenigen Mo, Fe und Bi enthaltenden Multimetalloxidaktivmassen, in deren Beisein das Cyclopropan in der ersten Reaktionsstufe in besonderer Weise für die unerwünschte Nebenreaktion empfänglich und bei deren Verwendung die erfindungsgemäße Verfahrensweise daher besonders relevant ist, lässt sich unter der allgemeinen Formel IV,

Mo₁₂BiₐFe_{b}X¹_{c}X²_{d}X³ₑX⁴_{f}Oₙ (IV),

in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,

a= 0,5 bis 5,
b= 0,01 bis 5, vorzugsweise 2 bis 4,
c= 0 bis 10, vorzugsweise 3 bis 10,
d= 0 bis 2, vorzugsweise 0,02 bis 2,
e= 0 bis 8, vorzugsweise 0 bis 5,
f= 0 bis 10 und
n= eine Zahl, die durch die Wertigkeit und Häufigkeit der von Sauerstoff verschiedenen Elemente in IV bestimmt wird,

### subsumieren.

Das Vorgenannte gilt vor allem, wenn sie in an sich bekannter Weise erhalten (siehe z. B. die DE-A 40 23 239) und z. B. in Substanz zu Kugeln, Ringen oder Zylindern geformt oder auch in Gestalt von Schalenkatalysatoren, d. h., mit der Aktivmasse beschichteten vorgeformten, inerten Trägerkörpern, erfindungsgemäß eingesetzt werden. Selbstverständlich gilt das Gesagte aber auch, wenn sie in Pulverform als Katalysatoren für die erste Reaktionsstufe angewendet werden (z. B. in Wirbelbettreaktoren).

Prinzipiell werden Aktivmassen der allgemeinen Formel IV in einfacher Weise in der Regel dadurch hergestellt, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für die erste Reaktionsstufe des erfindungsgemäßen Verfahrens sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Anstelle von Graphit kann aber auch hexagonales Bornitrid als Hilfsmittel bei der Formgebung verwendet werden, wie es die DE-A 10 2005 037 678 empfiehlt. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine erfindungsgemäß besonders relevante Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der erfindungsgemäß relevanten pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird häufig im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Erfindungsgemäß relevant ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Erfindungsgemäß relevant ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Erfindungsgemäß zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß relevant sind auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein erfindungsgemäß relevante Multimetalloxidaktivmassen sind ferner Massen der allgemeinen Formel V,

[Y¹_{a'}Y²_{b'}Oₓ]ₚ[Y³_{c'}Y⁴_{d'}Y⁵_{e'}Y⁶_{f'}Y⁷_{a'}Y²_{h'}O_{y'}]_{q} (V),

in der die Variablen folgende Bedeutung haben:
Y¹ = nur Wismut oder Wismut und wenigstens eines der Elemente Tellur, Antimon, Zinn und Kupfer,
Y² = Molybdän, oder Wolfram, oder Molybdän und Wolfram,
Y³ = ein Alkalimetall, Thallium und/oder Samarium,
Y⁴ = ein Erdalkalimetall, Nickel, Kobalt, Kupfer, Mangan, Zink, Zinn, Cadmium und/oder Quecksilber,
Y⁵ = Eisen oder Eisen und wenigstens eines der Elemente Chrom und Cer,
Y⁶= Phosphor, Arsen, Bor und/oder Antimon,
Y⁷ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiedenen Elemente in V bestimmt werden und
p,q = Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹ₐ'Y²_{b}'O_{x'}, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders relevante Multimetalloxidmassen V sind erfindungsgemäß solche, in denen Y¹ nur Wismut ist.

Unter diesen sind wiederum jene von besonderer Relevanz, die der allgemeinen Formel VI,

[Bi_{a"}Z²_{b"}O_{x"}]_{p"} [Z²1₂Z³_{c"}Z⁴_{d"}Fe_{e"}Z⁵_{f"}Z⁶_{g"}Z⁷_{h"}O_{y"}]_{q"} (VI),

in der die Variablen folgende Bedeutung haben:
Z² = Molybdän, oder Wolfram, oder Molybdän und Wolfram,
Z³ = Nickel und/oder Kobalt,
Z⁴ = Thallium, ein Alkalimetall und/oder ein Erdalkalimetall,
Z⁵ = Phosphor, Arsen, Bor, Antimon, Zinn, Cer und/oder Blei,
Z⁶ = Silicium, Aluminium, Titan und/oder Zirkonium,
Z⁷ = Kupfer, Silber und/oder Gold,
a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiedenen Element in VI bestimmt werden,
p",q"=Zahlen, deren Verhältnis p"/q" 0,1 bis 5, vorzugsweise 0,5 bis 2 beträgt,
entsprechen, wobei diejenigen Massen VI ganz besonders bevorzugt werden, in denen Z²_{b"} = (Wolfram)_{b"} und Z²₁₂ = (Molybdän)₁₂ ist.

Ferner ist es erfindungsgemäß von Bedeutung, wenn wenigstens 25 mol-% (bevorzugt wenigstens 50 mol-% und besonders bevorzugt 100 mol-%) des gesamten Anteils [Y¹_{a'}Y²_{b'x'}]ₚ ([Bi_{a"}Z²_{b"}O_{x"}]_{p"}) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a"}Z²_{b"}O_{x"}] vorliegen, deren Größtdurchmesser im Bereich 1 nm bis 100 µm liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

Für den zweiten Schritt (die zweite Reaktionsstufe), die heterogen katalysierte Gasphasen-Partialoxidation von Acrolein zu Acrylsäure, kommen erfindungsgemäß prinzipiell alle Mo und V enthaltenden Multimetalloxidmassen als Aktivmassen für die benötigten Katalysatoren in Betracht, z. B. jene der DE-A 100 46 928 und der DE-A 198 15 281.

Eine Vielzahl derselben, die für die unerwünschte Reaktion des Cyclopropan erfindungsgemäß besonders relevant sind, lässt sich unter der allgemeinen Formel VII,

Mo₁₂VₐX¹_{b}X²_{c}X³_{d}X⁴ₑX⁵_{f}X⁶_{g}Oₙ (VII),

in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,

a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in VII bestimmt wird,

### subsumieren.

Erfindungsgemäß besonders relevante Ausführungsformen innerhalb der aktiven Multimetalloxide VII sind jene, die von den nachfolgenden Bedeutungen der Variablen der allgemeinen Formel VII erfasst werden:
X¹ = W, Nb, und/oder Cr,
X² = Cu, Ni, Co, und/oder Fe,
X³ = Sb,
X⁴ = Na und/oder K,
X⁵ = Ca, Sr und/oder Ba,
X⁶ = Si, Al, und/oder Ti,

a = 1,5 bis 5,
b = 0,5 bis 2,
c = 0,5 bis 3,
d = 0 bis 2,
e = 0 bis 0,2,
f = 0 bis 1 und
n = eine Zahl, die durch die Wertigkeit und Häufigkeit der von Sauerstoff verschiedenen Elemente in VII bestimmt wird.

Erfindungsgemäß ganz besonders relevante Multimetalloxide VII sind jedoch jene der allgemeinen Formel VIII,

Mo₁₂V_{a'}Y¹_{b'}Y²_{c'}Y⁵_{f'}Y⁶_{g'}O_{n'} (VIII),

mit
Y¹ = W und/oder Nb,
Y² = Cu und/oder Ni,
Y⁵ = Ca und/oder Sr,
Y⁶ = Si und/oder Al,

a' = 2 bis 4,
b' = 1 bis 1,5,
c' = 1 bis 3,
f' = 0 bis 0,5
g' = 0 bis 8 und
n' = eine Zahl, die durch die Wertigkeit und Häufigkeit der von Sauerstoff verschiedenen Elementen in VIII bestimmt wird.

Die erfindungsgemäß relevanten Multimetalloxidaktivmassen (VII) sind in an sich bekannter, z. B. in der DE-A 43 35 973 oder in der EP-A 714 700 offenbarter, Weise erhältlich.

Generell können für den Schritt "Acrolein → Acrylsäure" erfindungsgemäß relevante Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel VII, können für die erfindungsgemäße Acroleinoxidation sowohl in Pulverform (z. B. in Wirbelbettreaktoren) als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Relevante Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird in erfindungsgemäß relevanter Weise häufig im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Relevant sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den erfindungsgemäßen Schritt "Acrolein → Acrylsäure" relevante Multimetalloxidaktivmassen sind ferner Massen der allgemeinen Formel IX,

[D]ₚ[E]_{q} (IX),

in der die Variablen folgende Bedeutung haben:
D = Mo₁₂V_{a"}Z¹_{b"}Z²_{c"}Z³_{d"}Z⁴_{e"}Z⁵_{f"}Z⁶_{g"}O_{x"},
E = Z⁷1₂Cu_{h"}H_{i"}O_{y"},
Z¹ = W, Nb, Ta, Cr und/oder Ce,
Z² = Cu, Ni, Co, Fe, Mn und/oder Zn,
Z³ = Sb und/oder Bi,
Z⁴ = Li, Na, K, Rb, Cs und/oder H
Z⁵ = Mg, Ca, Sr und/oder Ba,
Z⁶ = Si, Al, Ti und/oder Zr,
Z⁷ = Mo, W, V, Nb und/oder Ta, vorzugsweise Mo und/oder W

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in IX bestimmt werden und
p,q = von Null verschiedene Zahlen, deren Verhältnis p/q 160:1 bis 1:1 beträgt,
und die dadurch erhältlich sind, dass man eine Multimetalloxidmasse E

Z⁷₁₂Cu_{h"}H_{i"}O_{y"} (E),

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Besonders relevant sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein → Acrylsäure" erfindungsgemäß besonders relevante Multimetalloxidkatalysatoren sind auch jene der DE-A 198 15 281, insbesondere mit Multimetalloxidaktivmassen der allgemeinen Formel I dieser Schrift.

Mit erfindungsgemäßer Relevanz werden für den Schritt vom Propylen zum Acrolein Vollkatalysatorringe und für den Schritt vom Acrolein zur Acrylsäure Schalenkatalysatorringe verwendet.

Die Reaktionstemperatur in der ersten Reaktionsstufe beträgt erfindungsgemäß zweckmäßig 270 bis 450°C bzw. 280 bis 420°C, vorzugsweise 300 bis 380°C. Die Reaktionstemperatur in der zweiten Reaktionsstufe beträgt erfindungsgemäß zweckmäßig 200 bis 370 bzw. bis 320°C, vorzugsweise 220 bis 300°C.

Ferner ist das erfindungsgemäße Verfahren von besonderer Relevanz, wenn die Aktivmassen für die Katalysatoren der ersten Reaktionsstufe solche umfassen, deren spezifische Oberfläche 0,1 bis 120 m²/g, bzw. 0,2 bis 50 m²/g, bzw. 1 bis 20 m²/g, oder 2 bis 10 m²/g beträgt.

Auch ist das erfindungsgemäße Verfahren von besonderer Relevanz, wenn die Aktivmassen für die Katalysatoren der ersten Reaktionsstufe solche umfassen, deren numerisch häufigster Porendurchmesser 0,1 bis 1 µm beträgt.

Es ist weiterhin von besonderer Relevanz, wenn bei den Aktivmassen für die Katalysatoren der ersten Reaktionsstufe vorgenannter numerisch häufigster Porendurchmesser und eine der vorgenannten spezifischen Oberflächen kombiniert vorliegen.

Außerdem ist das erfindungsgemäße Verfahren von besonderer Bedeutung, wenn bei den Aktivmassen für die Katalysatoren der ersten Reaktionsstufe der Anteil der verschiedenen Porendurchmesser am Porengesamtvolumen wie folgt verteilt ist:
Poren mit Durchmesser im Bereich < 0,03 µm : ≥ 0 und ≤ 5 Vol.-%.
Poren mit Durchmesser im Bereich ≥ 0,003 µm bis ≤ 0,1 µm : ≥ 3 und ≤ 20 Vol.-%.

Poren mit Durchmesser im Bereich > 0,1 bis < 1 µm : ≥ 75 und ≤ 95 Vol.-% und Poren mit Durchmesser im Bereich ≥ 1 bis ≤ 10 µm : ≥ 0 und ≤ 5 Vol.-%.

Das Porengesamtvolumen beträgt für erfindungsgemäß relevante Erststufenkatalysatoraktivmassen typisch 0,1 bis 1,00 ml/g, meist 0,10 bis 0,80 ml/g, bzw. 0,20 bis 0,40 ml/g.

Weiterhin ist das erfindungsgemäße Verfahren von besonderer Relevanz, wenn die Aktivmasse für die Katalysatoren der zweiten Reaktionsstufe solche umfassen, deren spezifische Oberfläche 0,1 bis 150 m²/g, bzw. 0,2 bis 50 m²/g, bzw. 1 bis 20 m²/g oder 2 bis 10m²/g beträgt. Zusätzlich ist das erfindungsgemäße Verfahren von besonderer Relevanz, wenn die Aktivmassen für die Katalysatoren der zweiten Reaktionsstufe solche umfassen, deren numerisch häufigster Porendurchmesser 0,1 bis 1 µm beträgt.

Es ist weiteren von besonderer Relevanz, wenn bei den Aktivmassen für die Katalysatoren der zweiten Reaktionsstufe vorgenannter numerisch häufigster Porendurchmesser und eine der vorgenannten spezifischen Oberflächen kombiniert vorliegen.

Das Porengesamtvolumen beträgt für erfindungsgemäß relevante Zweitstufenkatalysatoren typisch 0,10 bis 0,90 ml/g, bzw. 0,20 bis 0,80 ml/g, oder 0,30 bis 0,70 ml/g.

Außerdem ist das erfindungsgemäße Verfahren von besonderer Bedeutung, wenn bei den Aktivmassen für die Katalysatoren der zweiten Reaktionsstufe die Porenverteilung so beschaffen ist, dass auf die Durchmesserbereiche 0 bis < 1,0 µm, 1,0 bis < 10 µm und 10 µm bis 100 µm jeweils wenigstens 5 Vol.-%, bevorzugt 10 Vol.-% des vorgenannten Porengesamtvolumens entfallen.

Vorteilhaft ist die erfindungsgemäße Verfahrensweise auch dann, wenn die Porendurchmesser-Verteilungen gemäß der EP-A 293 859 bei den Zweitstufenkatalysatoraktivmassen vorliegen. Jede einzelne der vorstehend gemachten Aussagen zu spezifischer Oberfläche, Porendurchmesser, Porengesamtvolumen und Porendurchmesser-Verteilung gilt insbesondere im Bezug auf jede einzelne in dieser Schrift als relevant erwähnte Multimetalloxidmasse für Katalysatoren der ersten Oxidationsstufe und der zweiten Oxidationsstufe.

Prinzipiell kann beim erfindungsgemäßen Verfahren innerhalb der ersten Reaktionsstufe die volumenspezifische Aktivität des wenigstens einen ersten Katalysatorbetts (insbesondere Katalysatorfestbetts) in Strömungsrichtung das Reaktionsgasgemischs 1 über die Länge des Strömungsweges entweder konstant sein, oder wenigstens einmal (kontinuierlich, oder abrupt oder stufenförmig) zunehmen. Dabei ist die wenigstens einmalige Zunahme unter den erfindungsgemäßen Gesichtspunkten (möglichst geringe Nebenproduktbildung) erfindungsgemäß bevorzugt. In allen vorgenannten Fällen ist es ferner von Vorteil, wenn sich die Aktivmassenzusammensetzung über die Länge des Strömungsweges innerhalb der ersten Reaktionsstufe nicht ändert.

Das für die erste Reaktionsstufe Vorgenannte gilt ebenso für die zweite Reaktionsstufe des erfindungsgemäßen Verfahrens.

Ist das Katalysatorbett für die erste Reaktionsstufe ein Katalysatorfestbett, so können zur Bereitung dieser Festbettkatalysatorschüttung 1 beim erfindungsgemäßen Verfahren nur Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden, Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit in Betracht.

Die Geometrie solcher inerter Verdünnungsformkörper kann im Prinzip beliebig sein. D.h., es können beispielsweise Kugeln, Polygone, Vollzylinder oder auch, wie die Aktivmasse aufweisenden Katalysatorformkörper, Ringe sein. Erfindungsgemäß bevorzugt wird man als inerte Verdünnungsformkörper solche wählen, deren Geometrie derjenigen der mit ihnen zu verdünnenden Katalysatorformkörper entspricht (vorgenannte Aussagen gelten so auch für zur Bereitung einer Festbettkatalysatorschüttung 2 (Katalysatorfestbett für die zweite Reaktionsstufe) verwendbare weitgehend homogene Gemische aus Multimetalloxidaktivmasse aufweisenden Katalysatorformkörpern und Verdünnungsformkörpern).

Vorteilhaft ist es, wenn sich die chemische Zusammensetzung der verwendeten Aktivmasse über die Festbettkatalysatorschüttung 1 nicht verändert. D.h., die für einen einzelnen Katalysatorformkörper verwendete Aktivmasse kann zwar ein Gemisch aus verschiedenen Multimetalloxiden sein, für alle Katalysatorformkörper der Festbettkatalysatorschüttung 1 ist dann jedoch das gleiche Gemisch zu verwenden.

Die volumenspezifische (d.h., die auf die Einheit des Volumens normierte) Aktivität kann in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen der Schüttung enthaltene Aktivmasse bzw. Katalysatoraktivität.

Eine in Strömungsrichtung des Reaktionsgasgemisches über die Festbettkatalysatorschüttung 1 wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Lässt man den Anteil an Verdünnungsformkörpern konstant oder werden überhaupt keine Verdünnungsformkörper in der Festbettkatalysatorschüttung 1 mitverwendet, resultiert eine konstante volumenspezifische Aktivität in Strömungsrichtung des Reaktionsgasgemisches über die Festbettkatalysatorschüttung 1. Eine Zunahme der volumenspezifischen Aktivität ist aber auch z.B. dadurch möglich, dass man bei gleichbleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Im Normalfall wird beim erfindungsgemäßen Verfahren weder innerhalb der Festbettkatalysatorschüttung 1 noch innerhalb der Festbettkatalysatorschüttung 2 in Strömungsrichtung des Reaktionsgasgemisches die volumenspezifische Aktivität einmal abnehmen.

Vorab und/oder im Anschluss an die Festbettkatalysatorschüttung 1 können sich ausschließlich aus Inertmaterial (z.B. nur Verdünnungsformkörpern) bestehende Schüttungen befinden (sie werden in dieser Schrift begrifflich nicht der Festbettkatalysatorschüttung 1 zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung 1 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Erfindungsgemäß bevorzugt ist beim erfindungsgemäßen Verfahren die Festbettkatalysatorschüttung 1 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h. z.B. auf einer Länge von 0,70 bis 1,50 m, bevorzugt 0,90 bis 1,20 m), jeweils der Gesamtlänge der Festbettkatalysatorschüttung 1 entweder nur Katalysatorformkörper, oder ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung 1 befindet sich dann erfindungsgemäß vorteilhaft bis zum Ende der Länge der Festbettkatalysatorschüttung 1 (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind.

Das Vorgenannte trifft insbesondere dann zu, wenn in der Festbettkatalysatorschüttung 1 als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

In entsprechender Weise wie die volumenspezifische Aktivität der Festbettkatalysatorschüttung 1 variiert werden kann, kann auch die volumenspezifische Aktivität der Festbettkatalysatorschüttung 2 variiert werden. Dabei kann sich vorab und/oder im Anschluss an die eigentliche Festbettkatalysatorschüttung 2 wiederum eine entsprechende Inertschüttung befinden.

Erfindungsgemäß bevorzugt ist beim erfindungsgemäßen Verfahren die Festbettkatalysatorschüttung 2 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Festbettkatalysatorschüttung 2, entweder nur Katalysatorformkörper oder ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 10 bis 50 Gew.-%, vorzugsweise 20 bis 45 Gew.-% und besonders bevorzugt 25 bis 35 Gew.-% beträgt. Im Anschluss an diese erste Zone der Festbettkatalysatorschüttung 2 befindet sich dann erfindungsgemäß vorteilhaft bis zum Ende der Länge der Festbettkatalysatorschüttung 2 (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn in der Festbettkatalysatorschüttung 2 als Katalysatorformkörper Schalenkatalysatorringe eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Anwendungstechnisch zweckmäßig kann die Durchführung der ersten Reaktionsstufe des erfindungsgemäßen Verfahrens z.B. in einem mit der Festbettkatalysatorschüttung 1 (sowie gegebenenfalls dieser vorangehenden und/oder nachfolgenden Inertschüttungen) beschickten Rohrbündelreaktor, wie er z.B. in der EP-B 700714 beschrieben ist, erfolgen.

D.h., in einfachster Weise befindet sich in den einzelnen Metallrohren eines Rohrbündelreaktors jeweils die vorgenannte Beschickung und um die Metallrohre wird ein Temperaturmedium (Einabschnittfahrweise), in der Regel eine Salzschmelze, geführt. Salzschmelze und Reaktionsgasgemisch können dabei im einfachen Gleich- oder Gegenstrom geführt werden. Die Salzschmelze (das Temperiermedium) kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemisches besteht. Die Strömungsgeschwindigkeit des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittsstelle in den Reaktor bis zur Austrittsstelle aus dem Reaktor ≥ 0 bis 10°C, häufig ≥ 2 bis 8°C, oft ≥ 3 bis 6°C beträgt. Die Eintrittstemperatur des Wärmeaustauschmittels in den Rohrbündelreaktor beträgt in der Regel 300 bis 360°C, häufig 300 bis 340°C.

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt (vor allem bei Anwendung der in dieser Schrift genannten Katalysatorringgeometrien) in der Regel 20 bis 30 mm, häufig 21 bis 26 mm. Ihre Länge liegt typisch bei 2 bis 4 m, häufig bei 2,5 bis 3,5 m. Davon werden erfindungsgemäß normalerweise wenigstens 60 %, häufig wenigstens 75 % von der Festbettkatalysatorschüttung 1 belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000, oder bis 40000. Rohrbündelreaktoren mit mehr als 50000 Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290). Einen für das erfindungsgemäße Verfahren geeigneten Rohrbündelreaktor offenbaren auch die DE-A 10131126, die DE-A 10137768, die DE-A 10135498 und die DE-A 10232967.

Zweckmäßigerweise wird das Reaktionsgasausgangsgemisch 1 der Festbettkatalysatorschüttung 1 auf die Reaktionstemperatur vorerwärmt zugeführt. Diesem Zweck kann z.B. eine einer Festbettkatalysatorschüttung vorausgehende Schüttung mit Inertmaterial dienen.

Selbstredend kann die erste Reaktionsstufe des erfindungsgemäßen Verfahrens auch in einem Zwei(oder mehr)abschnittrohrbündelreaktor durchgeführt werden, wie er z.B. in den DE-A's 19910508, 19948523, 19910506 und 19948241 beschrieben ist. Eine bevorzugte Variante eines erfindungsgemäß einsetzbaren Zweiabschnittrohrbündelreaktors offenbart die DE-C 2830765. Aber auch die in der DE-C 2513405, der US-A 3147084, der DE-A 2201528, der EP-A 383224 und der DE-A 2903218 offenbarten Zweiabschnittrohrbündelreaktoren sind für eine Durchführung der ersten Reaktionsstufe des erfindungsgemäßen Verfahrens geeignet.

D.h., in einfachster Weise befindet sich die erfindungsgemäß zu verwendende Festbettkatalysatorschüttung 1 (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß einen Reaktionsabschnitt. D.h., in einfachster Weise umströmt z.B. ein Salzbad A denjenigen Abschnitt der Rohre (den Reaktionsabschnitt A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (den Reaktionsabschnitt B), in welchem sich die oxidative Anschlussumsetzung des Propens (beim einfachen Durchgang) bis zum Erreichen eines Umsatzwertes von wenigstens 90 mol-% vollzieht (bei Bedarf können sich an die erfindungsgemäß anzuwendenden Reaktionsabschnitte A,B weitere Reaktionsabschnitte anschließen, die auf individuellen Temperaturen gehalten werden).

Anwendungstechnisch zweckmäßig umfasst die erste Reaktionsstufe des erfindungsgemäßen Verfahrens keine weiteren Reaktionsabschnitte. D.h., das Salzbad B umströmt zweckmäßig den Abschnitt der Rohre, in welchem sich die oxidative Anschlussumsetzung des Propens (beim einfachen Durchgang) bis zu einem Umsatzwert ≥ 90 mol-%, oder ≥ 92 mol-%, oder ≥ 94 mol-%, oder ≥ 96 mol-% oder mehr vollzieht.

Üblicherweise liegt der Beginn des Reaktionsabschnitts B hinter dem Heißpunktmaximum des Reaktionsabschnitts A. Das Heißpunktmaximum des Reaktionsabschnitts B liegt normalerweise unterhalb der Heißpunktmaximaltemperatur des Reaktionsabschnitts A.

Die beiden Salzbäder A,B können erfindungsgemäß relativ zur Strömungsrichtung des durch die Reaktionsrohre strömenden Reaktionsgasgemischs im Gleichstrom oder im Gegenstrom durch den die Reaktionsrohre umgebenden Raum geführt werden. Selbstverständlich kann erfindungsgemäß auch im Reaktionsabschnitt A eine Gleichströmung und im Reaktionsabschnitt B eine Gegenströmung (oder umgekehrt) angewandt werden.

Selbstverständlich kann man in allen vorgenannten Fallkonstellationen innerhalb des jeweiligen Reaktionsabschnitts der, relativ zu den Reaktionsrohren, erfolgenden Parallelströmung der Salzschmelze noch eine Querströmung überlagern, so dass der einzelne Reaktionsabschnitt einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird auch bei der Zweiabschnittfahrweise das Reaktionsgasausgangsgemisch 1 der Festbettkatalysatorschüttung 1 auf die Reaktionstemperatur vorerwärmt zugeführt.

Üblicherweise sind auch in den Zweiabschnittrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jedem Temperaturabschnitt belegt die Festbettkatalysatorschüttung 1 wenigstens 60 % bzw. wenigstens 75 %, oder wenigstens 90 % der Länge des Abschnitts. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000 oder bis 40000. Rohrbündelreaktoren mit einer oberhalb von 50000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweiabschnittrohrbündelreaktoren die Fließgeschwindigkeit innerhalb der beiden erforderlichen Wärmeaustauschmittelkreisläufe so gewählt, dass die Temperatur des Wärmeaustauschmittels von der Eintrittsstelle in den Reaktionsabschnitt bis zur Austrittstelle aus dem Reaktionsabschnitt (bedingt durch die Exothermie der Reaktion) um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in den Reaktionsabschnitt A beträgt erfindungsgemäß normalerweise 300 bis 340°C. Die Eintrittstemperatur des Wärmeaustauschmittels in den Reaktionsabschnitt B beträgt erfindungsgemäß normalerweise einerseits 305 bis 380°C und liegt andererseits gleichzeitig wenigstens ≥ 0°C, oder wenigstens 5°C oberhalb der Eintrittstemperatur des in den Reaktionsabschnitt A eintretenden Wärmeaustauschmittels. Gegebenenfalls kann dieser Temperaturunterschied auch ≤ 0°C betragen.

Bei hohen Propenlasten liegt die Eintrittstemperatur des Wärmeaustauschmittels in den Reaktionsabschnitt B zweckmäßig wenigstens 10°C oberhalb der Eintrittstemperatur des in den Reaktionsabschnitt A eintretenden Wärmeaustauschmittels. Die Differenz zwischen den Eintrittstemperaturen in den Reaktionsabschnitt A bzw. B kann erfindungsgemäß somit bis zu 20°C, bis zu 25°C, bis zu 30°C, bis zu 40°C, bis zu 45°C oder bis zu 50°C betragen. Normalerweise wird die vorgenannte Temperaturdifferenz aber nicht mehr als 50°C betragen. Je höher die Propenbelastung der Festbettkatalysatorschüttung 1 beim erfindungsgemäßen Verfahren gewählt wird, um so größer sollte die Differenz zwischen der Eintrittstemperatur des Wärmeaustauschmittels in den Reaktionsabschnitt A und der Eintrittstemperatur des Wärmeaustauschmittels in den Reaktionsabschnitt B sein.

Mit Vorteil beträgt die Eintrittstemperatur des Wärmeaustauschmittels in den Reaktionsabschnitt B erfindungsgemäß 330 bis 370°C und besonders vorteilhaft 340 bis 370°C.

Selbstverständlich können beim erfindungsgemäßen Verfahren die beiden Reaktionsabschnitte A, B auch in räumlich voneinander getrennten Rohrbündelreaktoren realisiert sein. Bei Bedarf kann zwischen den beiden Reaktionsabschnitten A, B auch ein Wärmetauscher angebracht werden.

Es sei an dieser Stelle auch noch einmal darauf hingewiesen, dass für eine Durchführung der Reaktionsstufe 1 des erfindungsgemäßen Verfahrens insbesondere auch der in der DE-AS 2201528 beschriebene Zweiabschnittrohrbündelreaktortyp verwendet werden kann, der die Möglichkeit beinhaltet, vom heißeren Wärmeaustauschmittel des Reaktionsabschnitts B eine Teilmenge an den Reaktionsabschnitt A abzuführen, um gegebenenfalls ein Anwärmen eines kalten Reaktionsgasausgangsgemisches oder eines kalten Kreisgases zu bewirken. Ferner kann die Rohrbündelcharakteristik innerhalb eines individuellen Reaktionsabschnitts wie in der EP-A 382098 beschrieben gestaltet werden.

Erfindungsgemäß hat es sich als zweckmäßig erwiesen, das die erste Reaktionsstufe verlassende Produktgasgemisch vor dem Eintritt in die zweite Reaktionsstufe abzukühlen, um so eine Nachvollverbrennung von Teilen des in der ersten Reaktionsstufe gebildeten Acroleins zu unterdrücken. Üblicherweise wird dazu zwischen die beiden Reaktionsstufen ein Nachkühler geschaltet. Dies kann im einfachsten Fall ein indirekter Rohrbündelwärmeüberträger sein. Das Produktgasgemisch wird dabei in der Regel durch die Rohre geführt und um die Rohre wird ein Wärmetauschermedium geführt, dessen Art der für die Rohrbündelreaktoren empfohlenen Wärmetauschermedien entsprechen kann. Mit Vorteil ist das Rohrinnere mit inerten Füllkörpern (z.B. Spiralen aus Edelstahl, Ringe aus Steatit, Kugeln aus Steatit etc.) gefüllt. Selbige verbessern den Wärmeaustausch und fangen gegebenenfalls aus der Festbettkatalysatorschüttung der ersten Reaktionsstufe sublimierendes Molybdäntrioxid vor einem Eintritt desselben in die zweite Reaktionsstufe ab. Es ist von Vorteil, wenn der Nachkühler aus mit Zinksilicatfarbe beschichtetem rostfreiem Stahl gefertigt ist.

Die in der ersten Reaktionsstufe bei einfachem Durchgang resultierende Selektivität S^{AA} der Acroleinbildung sowie der Acrylsäurenebenproduktbildung wird (mit steigendem Propylenumsatz nimmt S^{AA} bei ansonsten gleichbleibenden Bedingungen teilweise leicht ab, da höhere Umsätze normalerweise höhere Reaktionstemperaturen erfordern) erfindungsgemäß zusammen regelmäßig ≥ 85 mol-%, bzw. ≥ 90 mol-%, vielfach ≥ 92 mol-% oder ≥ 94 mol-%, häufig ≥ 95 mol-% oder ≥ 96 mol-%, bzw. ≥ 97 mol-% betragen.

Das erfindungsgemäße Verfahren eignet sich für Propenbelastungen der Festbettkatalysatorschüttung 1 von ≥ 80 Nl/l·h, oder von ≥ 100 Nl/l·h, oder von ≥ 120 Nl/l·h, oder von ≥ 140 Nl/l·h, oder von ≥ 165 Nl/l·h, oder von ≥ 170 Nl/l·h bzw. ≥ 175 Nl/l·h oder ≥ 180 Nl/l·h, aber auch für Propenbelastungen der Festbettkatalysatorschüttung 1 von ≥ 185 Nl/l·h, oder ≥ 190 Nl/l·h bzw. ≥ 200 Nl/l·h oder ≥ 210 Nl/l·h sowie für Belastungswerte von ≥ 220 Nl/l·h oder ≥ 230 Nl/l·h bzw. ≥ 240 Nl/l·h oder ≥ 250 Nl/l·h.

Mit zunehmender Propenbelastung ist die beschriebene Zweiabschnittfahrweise gegenüber der beschriebenen Einabschnittsfahrweise in der ersten Reaktionsstufe bevorzugt.

Normalerweise wird beim erfindungsgemäßen Verfahren die Propenbelastung der ersten Festbettkatalysatorschüttung 600 Nl/l·h nicht überschreiten. In typischer Weise liegen die Propenbelastungen der Festbettkatalysatorschüttung 1 beim erfindungsgemäßen Verfahren bei Werten ≤ 300 Nl/l·h, häufig bei Werten ≤ 250 Nl/l·h.

Der Arbeitsdruck kann beim erfindungsgemäßen Verfahren in der ersten Reaktionsstufe sowohl unterhalb von Normaldruck (z.B. bis zu 0,5 bar, das Reaktionsgasgemisch wird durchgesaugt) als auch oberhalb von Normaldruck liegen. Typischerweise wird der Arbeitsdruck in der ersten Reaktionsstufe bei Werten von 1 bis 5 bar, häufig 1,5 bis 3,5 bar liegen. Normalerweise wird der Reaktionsdruck in der ersten Reaktionsstufe 100 bar nicht überschreiten.

Als Quelle für den in der ersten Reaktionsstufe erforderlichen molekularen Sauerstoff kommt sowohl Luft, als auch an molekularem Stickstoff entreicherte Luft in Betracht.

Anwendungstechnisch zweckmäßig wird das Produktgasgemisch der ersten Reaktionsstufe im bereits erwähnten Nachkühler auf eine Temperatur von 210 bis 290°C, häufig 230 bis 280°C oder 250 bis 270°C abgekühlt. Dabei kann die Abkühlung des Produktgasgemisches der ersten Reaktionsstufe durchaus auf Temperaturen erfolgen, die unterhalb der Temperatur der zweiten Reaktionsstufe liegen. Die beschriebene Nachkühlung ist jedoch keineswegs zwingend und kann insbesondere dann in aller Regel entfallen, wenn der Weg des Produktgasgemisches von der ersten Reaktionsstufe in die zweite Reaktionsstufe kurz gehalten wird. Üblicherweise wird das erfindungsgemäße Verfahren ferner so verwirklicht, dass man den Sauerstoffbedarf in der zweiten Reaktionsstufe nicht bereits durch einen entsprechend hohen Sauerstoffgehalt des Reaktionsgasausgangsgemisches 1 deckt, sondern den benötigten Sauerstoff im Bereich zwischen erster und zweiter Reaktionsstufe zugibt. Dies kann vor, während, nach und/oder zur Nachkühlung erfolgen. Als Quelle für den in der zweiten Reaktionsstufe erforderlichen molekularen Sauerstoff kommen sowohl reiner Sauerstoff als auch Gemische aus Sauerstoff und Inertgas, z.B. Luft oder an molekularem Stickstoff entreicherte Luft (z.B. ≥ 90 Vol-% O₂, ≤ 10 Vol-% N₂) in Betracht. Die Zugabe der Sauerstoffquelle erfolgt regelmäßig in auf den Reaktionsdruck komprimierter Form. Selbstredend kann beim erfindungsgemäßen Verfahren der Sauerstoffbedarf in der zweiten Reaktionsstufe bereits durch einen entsprechend hohen Sauerstoffbedarf in der ersten Reaktionsstufe gedeckt werden.

Der Arbeitsdruck kann beim erfindungsgemäßen Verfahren in der zweiten Reaktionsstufe wie in der Reaktionsstufe 1 sowohl unterhalb von Normaldruck (z.B. bis zu 0,5 bar) als auch oberhalb von Normaldruck liegen. Typischerweise wird der Arbeitsdruck in der zweiten Reaktionsstufe erfindungsgemäß bei Werten von 1 bis 5 bar, häufig 1 bis 3 bar liegen. Normalerweise wird der Reaktionsdruck in der zweiten Reaktionsstufe 100 bar nicht überschreiten.

Ebenso wie die erste Reaktionsstufe kann die zweite Reaktionsstufe des erfindungsgemäßen Verfahrens in einfacher Weise in einem mit der Festbettkatalysatorschüttung 2 beschickten Rohrbündelreaktor, wie er z.B. in der EP-A 700893 beschrieben ist, durchgeführt werden. Der Festbettkatalysatorschüttung 2 vorausgehende und/oder nachfolgende Inertschüttungen können die Beschickung ergänzen.

D.h., in einfachster Weise befindet sich der erfindungsgemäß zu verwendende Festbettkatalysator 2 sowie die gegebenenfalls mitverwendeten Inertschüttungen in den Metallrohren eines Rohrbündelreaktors und um die Metallrohre wird ein Temperiermedium (Einabschnittfahrweise), in der Regel eine Salzschmelze geführt. Salzschmelze und Reaktionsgasgemisch können dabei im einfachen Gleich- oder Gegenstrom geführt werden. Das Temperiermedium kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemisches besteht. Der Volumenstrom des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittstelle in den Reaktor bis zur Austrittstelle aus dem Reaktor ≥ 0 bis 10°C, häufig ≥ 2 bis 8°C, oft ≥ 3 bis 6°C beträgt. Die Eintrittstemperatur des Wärmeaustauschmittels in den Rohrbündelreaktor beträgt in der Regel 230 bis 300°C, häufig 245 bis 285°C bzw. 255 bis 275°C. Als Wärmeaustauschmittel eignen sich dabei die gleichen fluiden Temperiermedien, wie sie bereits für die erste Reaktionsstufe beschrieben worden sind.

Zweckmäßigerweise wird das Reaktionsgasausgangsgemisch 2 der Festbettkatalysatorschüttung 2 auf die Reaktionstemperatur vorerwärmt zugeführt. Für die Dimensionierung der Kontaktrohre, das Kontaktrohrmaterial, die Kontaktrohranzahl und ihre Beschickung mit Festbettkatalysatorschüttung 2/Inertschüttung gilt das für den Rohrbündelreaktor der ersten Reaktionsstufe Gesagte.

In der Regel wird eine Einabschnittfahrweise der ersten Reaktionsstufe mit einer Einabschnittfahrweise der zweiten Reaktionsstufe kombiniert, wobei die relative Stromführung von Reaktionsgasgemisch und Temperiermedium in beiden Stufen identisch gewählt wird.

Selbstverständlich kann aber auch die zweite Reaktionsstufe des erfindungsgemäßen Verfahrens in entsprechender Weise wie die erste Reaktionsstufe als zwei räumlich aufeinander folgende Reaktionsabschnitte C, D realisiert werden, wobei die Temperatur des Reaktionsabschnitts C (gemeint ist damit stets die Temperatur des eintretenden Salzbades bzw. allgemein Wärmeträgers) zweckmäßig 230 bis 270°C und die Temperatur des Reaktionsabschnitts D 250 bis 300°C beträgt und gleichzeitig wenigstens ≥ 0°C, oder wenigstens ≥ 5°C oberhalb der Temperatur der Reaktionszone C liegt. Gegebenenfalls kann dieser Temperaturunterschied aber auch ≤ 0°C betragen.

Bevorzugt erstreckt sich der Reaktionsabschnitt C bis zu einem Acroleinumsatz von 65 bis 80 mol-%. Außerdem liegt die Temperatur des Reaktionsabschnitts C mit Vorteil bei 245 bis 260°C. Die Temperatur des Reaktionsabschnitts D liegt bei hohen Acroleinlasten vorzugsweise 5 bis 10°C oberhalb der Temperatur des Reaktionsabschnitts C und beträgt vorteilhaft 260 bis 285°C. Auch für die Zweiabschnittfahrweise der zweiten Reaktionsstufe gilt bezüglich des Reaktors für die Dimensionierung der Kontaktrohre, das Kontaktrohrmaterial, die Kontaktrohranzahl und ihre Beschickung mit Festbettkatalysator 2/Inertschüttung das für den Zweiabschnittrohrbündelreaktor der ersten Reaktionsstufe Gesagte.

Je höher die Acroleinbelastung der Festbettkatalysatorschüttung 2 beim erfindungsgemäßen Verfahren gewählt wird, um so mehr ist die Zweiabschnittfahrweise gegenüber der Einabschnittfahrweise bevorzugt und um so größer sollte die Differenz zwischen der Temperatur des Reaktionsabschnitts C und der Temperatur des Reaktionsabschnitts D gewählt werden. Normalerweise wird die vorgenannte Temperaturdifferenz aber nicht mehr als 40°C betragen. D.h., die Differenz zwischen der Temperatur des Reaktionsabschnitts C und der Temperatur des Reaktionsabschnitts D kann erfindungsgemäß bis zu 15°C, bis zu 25°C, bis zu 30°C, bis zu 35°C oder bis zu 40°C betragen.

Die Selektivität der Acrylsäurebildung, bezogen auf umgesetztes Acrolein, wird beim erfindungsgemäßen Verfahren regelmäßig ≥ 90 mol-%, oder ≥ 92 mol-%, bzw. ≥ 94 mol-%, häufig ≥ 95 mol-% oder ≥ 96 mol-%, bzw. ≥ 97 mol-% betragen.

Das erfindungsgemäße Verfahren eignet sich für Acroleinbelastungen der Festbettkatalysatorschüttung 2 von ≥ 80 Nl/l·h, oder von ≥ 100 Nl/l·h, oder von ≥ 120 Nl/l·h, oder von ≥ 140 Nl/l·h bzw. ≥ 150 Nl/l·h, oder von ≥ 160 Nl/l·h bzw. ≥ 170 Nl/l·h, oder ≥ 175 Nl/l·h bzw. ≥ 180 Nl/l·h, aber auch bei Acroleinbelastungen der Festbettkatalysatorschüttung 2 von ≥ 185 Nl/l·h, oder von ≥ 190 Nl/l·h bzw. ≥ 200 Nl/l·h, oder ≥ 210 Nl/l·h sowie bei Belastungswerten ≥ 220 Nl/l·h, oder ≥ 230 Nl/l·h bzw. 240 Nl/l·h, oder ≥ 250 Nl/l·h.

Erfindungsgemäß bevorzugt, wird zwischen der ersten und der zweiten Reaktionsstufe kein nur aus Inertgas bestehendes Sekundärgas zudosiert.

Normalerweise wird beim erfindungsgemäßen Verfahren die Acroleinbelastung der zweiten Festbettkatalysatorschüttung den Wert von 600 Nl/l·h nicht überschreiten. In typischer Weise liegen die Acroleinbelastungen der Festbettkatalysatorschüttung 2 beim erfindungsgemäßen Verfahren ohne nennenswerten Verlust von Umsatz und Selektivität bei Werten ≤ 300 Nl/l·h, häufig bei Werten ≤ 250 Nl/l·h.

In der Regel wird beim erfindungsgemäßen Verfahren die Acroleinbelastung der Festbettkatalysatorschüttung 2 etwa 10 Nl/l·h, häufig etwa 20 bzw. 25 Nl/l·h unterhalb der Propenbelastung der Festbettkatalysatorschüttung 1 liegen. Dies ist primär darauf zurückzuführen, dass in der ersten Reaktionsstufe sowohl Umsatz als auch Selektivität zu Acrolein in der Regel nicht 100 % erreichen. Ferner wird der Sauerstoffbedarf der zweiten Reaktionsstufe üblicherweise durch Luft als Sekundärgas gedeckt. Mit zunehmender Acroleinbelastung ist die beschriebene Zweiabschnittfahrweise gegenüber der ausgeführten Einabschnittfahrweise in der zweiten Reaktionsstufe bevorzugt.

Beachtenswerterweise kann beim erfindungsgemäßen Verfahren die über beide Reaktionsstufen bilanzierte Selektivität der Acrylsäurebildung, bezogen auf umgesetztes Propen, selbst bei höchsten Propen- und Acroleinbelastungen in der Regel bei Werten ≥ 83 mol-%, häufig bei ≥ 85 mol-% oder ≥ 88 mol-%, oft bei ≥ 90 mol-% oder ≥ 93 mol-% oder mehr liegen.

In anwendungstechnisch zweckmäßiger Weise erfolgt die Durchführung der zweiten Reaktionsstufe des erfindungsgemäßen Verfahrens in einem Zweiabschnittrohrbündelreaktor. Eine bevorzugte Variante eines für die zweite Reaktionsstufe erfindungsgemäß einsetzbaren Zweiabschnittrohrbündelreaktors offenbart die DE-C 2830765. Aber auch die in der DE-C 2513405, der US-A 3147084, der DE-A 2201528, der EP-A 383224 und der DE-A 2903582 offenbarten Zweiabschnittrohrbündelreaktoren sind für eine Durchführung der zweiten Reaktionsstufe des erfindungsgemäßen Verfahrens geeignet.

D.h., in einfacher Weise befindet sich die erfindungsgemäß zu verwendende Festbettkatalysatorschüttung 2 (gegebenenfalls einschließlich der Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß einen Reaktionsabschnitt.

D.h., in einfacher Weise umströmt z.B. ein Salzbad C diejenigen Abschnitte der Rohre (den Reaktionsabschnitt C), in welchem sich die oxidative Umsetzung des Acroleins (beim einfachen Durchgang) bis zum Erreichen eines Umsatzwertes im Bereich von 55 bis 85 mol.-% vollzieht und ein Salzbad D umströmt den Abschnitt der Rohre (den Reaktionsabschnitt D), in welchem sich die oxidative Anschlussumsetzung des Acroleins (beim einfachen Durchgang) bis zum Erreichen eines Umsatzwertes von wenigstens 90 mol.-% vollzieht (bei Bedarf können sich an die erfindungsgemäß anzuwendenden Reaktionszonen C,D weitere Reaktionszonen anschließen, die auf individuellen Temperaturen gehalten werden).

Anwendungstechnisch zweckmäßig umfasst die Reaktionsstufe 2 des erfindungsgemäßen Verfahrens keine weiteren Reaktionsabschnitte. D.h., das Salzbad D umströmt zweckmäßig den Abschnitt der Rohre, in welchem sich die oxidative Anschlussumsetzung des Acroleins (beim einfachen Durchgang) bis zu einem Umsatzwert von ≥ 92 mol.-%, oder ≥ 94 mol.-%, oder ≥ 96 mol.-%, oder ≥ 98 mol.-%, und häufig sogar ≥ 99 mol.-% oder mehr vollzieht.

Üblicherweise liegt der Beginn des Reaktionsabschnitts D hinter dem Heißpunktmaximum des Reaktionsabschnitts C. Die Temperatur des Heißpunktmaximums des Reaktionsabschnitts D liegt normalerweise unterhalb der Heißpunktmaximaltemperatur des Reaktionsabschnitts C.

Die beiden Salzbäder C, D können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch im Reaktionsabschnitt C eine Gleichströmung und im Reaktionsabschnitt D eine Gegenströmung (oder umgekehrt) angewandt werden.

Selbstredend kann man in allen vorgenannten Fallkonstellationen innerhalb des jeweiligen Reaktionsabschnitts der, relativ zu den Reaktionsrohren, erfolgenden Parallelströmung der Salzschmelze noch eine Querströmung überlagern, so dass der einzelne Reaktionsabschnitt einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Üblicherweise sind in den vorgenannten Zweizonen-Rohrbündelreaktoren (ebenso wie in den Rohrbündelreaktoren der Einzonenfahrweise) die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Ihre Länge beträgt zweckmäßig 3 bis 4, bevorzugt 3,5 m. In jeder Temperaturzone belegt die Festbettkatalysatorschüttung 2 wenigstens 60 %, bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000 oder 40000. Rohrbündelreaktoren mit einer oberhalb von 50000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweiabschnittrohrbündelreaktoren die Fließgeschwindigkeit innerhalb der beiden erforderlichen Wärmeaustauschmittelkreisläufe so gewählt, dass die Temperatur des Wärmeaustauschmittels von der Eintrittstelle in die Reaktionszone bis zur Austrittsstelle aus der Reaktionszone um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in den Reaktionsabschnitt C beträgt bei einer erfindungsgemäßen Zweiabschnittfahrweise in der zweiten Reaktionsstufe normalerweise 230 bis 270°C. Die Eintrittstemperatur des Wärmeaustauschmittels in den Reaktionsabschnitt D beträgt dabei erfindungsgemäß normalerweise einerseits 250 bis 300°C und liegt andererseits gleichzeitig wenigstens ≥ 0°C, oder wenigstens ≥ 5°C oberhalb der Eintrittstemperatur des in den Reaktionsabschnitt C eintretenden Wärmeaustauschmittels.

Bei hohen Acroleinlasten liegt die Eintrittstemperatur des Wärmeaustauschmittels in den Reaktionsabschnitt D bevorzugt 5 bis 10°C oberhalb der Eintrittstemperatur des in den Reaktionsabschnitt C eintretenden Wärmeaustauschmittels. Die Differenz zwischen den Eintrittstemperaturen in den Reaktionsabschnitt C bzw. D kann erfindungsgemäß aber auch bis zu 15°C, bis zu 25°C, bis zu 30°C, bis zu 35°C oder bis zu 40°C betragen. Normalerweise wird die vorgenannte Temperatur aber nicht mehr als 50°C betragen. Je höher die Acroleinbelastung der Katalysatorschüttung 2 beim erfindungsgemäßen Verfahren gewählt wird, um so größer sollte die Differenz zwischen der Eintrittstemperatur des Wärmeaustauschmittels in den Reaktionsabschnitt C und der Eintrittstemperatur des Wärmeaustauschmittels in den Reaktionsabschnitt D sein. Bevorzugt liegt die Eintrittstemperatur des Wärmeaustauschmittels in den Reaktionsabschnitt C bei 245 bis 260°C und die Eintrittstemperatur in den Reaktionsabschnitt D bei 260 bis 285°C.

Selbstverständlich können beim erfindungsgemäßen Verfahren die beiden Reaktionsabschnitte C, D auch in räumlich voneinander getrennten Rohrbündelreaktoren realisiert sein. Bei Bedarf kann zwischen den beiden Reaktionsabschnitten C, D auch ein Wärmetauscher angebracht werden.

Es sei an dieser Stelle auch noch einmal darauf hingewiesen, dass für eine Durchführung der zweiten Reaktionsstufe des erfindungsgemäßen Verfahrens insbesondere auch der in der DE-AS 2201528 beschriebene Zweiabschnittrohrbündelreaktortyp verwendet werden kann, der die Möglichkeit beinhaltet, vom heißeren Wärmeaustauschmittel des Reaktionsabschnitts D eine Teilmenge an den Reaktionsabschnitt C abzuführen, um gegebenenfalls ein Anwärmen eines zu kalten Reaktionsgasausgangsgemisches 2 oder eines kalten Kreisgases zu bewirken. Ferner kann die Rohrbündelcharakteristik innerhalb eines individuellen Reaktionsabschnitts wie in der EP-A 382 098 beschrieben gestaltet werden.

Selbstredend können beim erfindungsgemäßen Verfahren zwei Einabschnittrohrbündelreaktoren für die beiden Reaktionsstufen auch zu einem auf andere Art und Weise zu betreibenden einzelnen Zweiabschnittreaktor verschmolzen werden, wie es z. B. in der DE-C 2830765, in der EP-A 911313 sowie in der EP-A 383 224 beschrieben ist. In diesem Fall wird die erste Reaktionsstufe im ersten Reaktionsabschnitt und die zweite Reaktionsstufe im zweiten Reaktionsabschnitt des Zweizonenrohrbündelreaktors verwirklicht.

In völliger Entsprechung können auch ein Einabschnittrohrbündelreaktor und ein Zweiabschnittrohrbündelreaktor oder zwei Zweiabschnittrohrbündelreaktoren zu jeweils einem einzigen Rohrbündelreaktor verschmolzen werden, der dann drei bzw. vier Temperaturabschnitte aufweist und z. B. in der WO 01/36364 beschrieben ist.

In diesem Fall kann z. B. die erste Reaktionsstufe im ersten Reaktionsabschnitt und die zweite Reaktionsstufe in den beiden nachfolgenden Reaktionsabschnitten des Dreiabschnittrohrbündelreaktors durchgeführt werden. Alternativ kann z. B. die erste Reaktionsstufe in den ersten beiden Reaktionsabschnitten und die zweite Reaktionsstufe in den beiden nachfolgenden Reaktionsabschnitten des Vierabschnittrohrbündelreaktors durchgeführt werden u.s.w.. Die Salzbadtemperatur der einzelnen Temperaturabschnitte kann dabei wie im Fall der voneinander räumlich getrennten Rohrbündelreaktoren beschrieben gestaltet werden. Normalerweise befindet sich in diesen Fällen zwischen der Festbettkatalysatorschüttung 1 und der Festbettkatalysatorschüttung 2 eine Inertschüttung. Allerdings kann auf eine solche Zwischeninertschüttung auch verzichtet werden. Die Länge der Reaktionsrohre entspricht in den Verschmelzungsfällen vielfach der Summe der Längen der nicht verschmolzenen Rohrbündelreaktoren. Selbstredend kann das erfindungsgemäße Verfahren auch analog zur in den Schriften EP-A 990636 und EP-A 1 106 598 beschriebenen Verfahrensweisen ausgeführt werden.

In der Regel enthält das Reaktionsgasausgangsgemisch 1 beim erfindungsgemäßen Verfahren 3 bis 25 Vol.-%, vielfach 5 bis 20 Vol.-%, und meist 6 bis 13 Vol.-%, Propylen.

Der Gehalt an molekularem Sauerstoff im Reaktionsgasausgangsgemisch 1 ist erfindungsgemäß so zu bemessen, dass das molare Verhältnis V₁ von im Reaktionsgasausgangsgemisch 1 enthaltenem O₂ zu im Reaktionsgasausgangsgemisch enthaltenem C₃H₆ ≥ 1 beträgt. Üblicherweise beträgt V₁ beim erfindungsgemäßen Verfahren ≥ 1 und ≤ 3, meist ≥ 1,3 und ≤ 2,5, oft ≥ 1,5 bis ≤ 2,3. Die Menge an molekularem Sauerstoff im Reaktionsgasausgangsgemisch 2 wird normalerweise so bemessen, dass das molare Verhältnis von im Reaktionsgasausgangsgemisch 2 enthaltenem O₂ zu im Reaktionsgasausgangsgemisch 2 enthaltenem Acrolein ≥ 0,5 bis ≤ 2, häufig ≥ 0,75 bis ≤ 1,5 beträgt. Günstig ist es, wenn das Produktgasgemisch 2 noch bis zu 5, bzw. bis zu 3 Vol.-% molekularen Sauerstoff enthält.

Auch kann das Reaktionsgasausgangsgemisch 1 ≥ 0,01, oder ≥ 0,1, oder ≥ 0,5, oder ≥ 2 Vol.-% an CO₂ enthalten. Meist kann der vorgenannten CO₂-Gehalt ≤ 25 Vol.-% betragen.

Insbesondere dann, wenn als Quelle für den molekularen Sauerstoff beim erfindungsgemäßen Verfahren Luft verwendet wird, wird das Reaktionsgasausgangsgemisch 1 als weiteres inertes Verdünnungsgas molekularen Stickstoff enthalten. Grundsätzlich kann das Reaktionsgasausgangsgemisch 1 beim erfindungsgemäßen Verfahren ≥ 1 Vol.-%, oder ≥ 5 Vol.-%, oder ≥ 10 Vol.-%, oder ≥ 20 Vol.-%, oder ≥ 30 Vol.-%, oder ≥ 40 Vol.-% an molekularem Stickstoff enthalten. In der Regel wird der Gehalt des Reaktionsgasausgangsgemischs 1 an molekularem Stickstoff jedoch bei Werten ≤ 80 mol-%, oder ≤ 70 mol-%, oder ≤ 60 mol-% liegen.

Erfindungsgemäß wesentlich, muss das Reaktionsgasausgangsgemisch 1 Propan als inertes Verdünnungsgas enthalten. Dieser Propangehalt des Reaktionsgasausgangsgemischs 1 kann bis zu 70 Vol.-% (z. B. 5 bis 70 Vol.-%), bzw. bis zu 60 Vol.-%, oder bis 50 Vol.-%, oder bis zu 40 Vol.-%, oder bis 30 Vol.-%, oder bis 20 Vol.-%, oder bis zu 10 Vol.-% betragen. Häufig liegt dieser Propangehalt bei ≥ 0,5 bzw. ≥ 1 Vol.-%. Er kann aber auch bei Werten von ≥ 0,01 Vol.-%, oder ≥ 0,02 Vol.-%, oder ≥ 0,03 Vol.-% liegen. In der Regel enthält das Reaktionsgasausgangsgemisch 1 ≤ 10 Vol.-%, vielfach ≤ 5 Vol.-% an Propan.

Dieses Propan kann beim erfindungsgemäßen Verfahren z. B. bewusst als dem Reaktionsgasausgangsgemisch 1 separat zuzuführendes inertes Verdünnungsgas zugesetzt werden. Normalerweise wird dies in Form von Roh-Propan erfolgen, das von Natur aus auch Cyclopropan enthalten kann. Wie im Fall von Roh-Propylen kann, um das anspruchsgemäße Ziel für das Reaktionsgasausgangsgemisch 1 zu erreichen, auch hier im Roh-Propan enthaltenes Cyclopropan vorab einer Verwendung des Roh-Propan für das erfindungsgemäße Verfahren rektifikativ abgetrennt oder durch überleiten des Roh-Propan über geeignete Katalysatoren in Propylen gewandelt werden.

Selbstverständlich kann das Propan aber auch dadurch Bestandteil das Reaktionsgasausgangsgemisches 1 werden, dass als Propylenquelle für selbiges eine partielle Dehydrierung oder Oxidehydrierung von Propan fungiert (in der Regel werden diese heterogen katalysiert erfolgen). D. h., das im Reaktionsgasausgangsgemisch 1 enthaltene Propylen kann dem Reaktionsgasausgangsgemisch 1 wenigstens teilweise unter Begleitung von nicht umgesetztem Propan aus einer partiellen Dehydrierung (z. B. homogen und/oder heterogen katalysiert, im Beisein und/oder unter Ausschluss von molekularem Sauerstoff) zugeführt werden. Erfindungsgemäß zweckmäßig wird man das für eine solche partielle Dehydrierung zu verwendende Roh-Propan vorab gleichfalls einer Cyclopropaneliminierung unterwerfen.

Grundsätzlich können die Gehalte des Reaktionsgasausgangsgemischs 1 und von Roh-Gasen problemlos gaschromatographisch ermittelt werden. Durch Analyse von kondensierter Phase des Reaktionsgasausgangsgemischs 1 ist die Nachweisgrenze für Cyclopropan und andere C₃-Kohlenwasserstoffe erweiterbar.

Besonders relevant ist die erfindungsgemäße Verfahrensweise dann, wenn das Reaktionsgasausgangsgemisch 1 Wasserdampf enthält, da dieser die Wandlung des Cyclopropan fördert.

Das erfindungsgemäße Verfahren umfasst daher insbesondere solche Ausführungsformen, bei denen das Reaktionsgasausgangsgemisch 1 > 0 bis 35 Vol.-%, häufig 1 bis 25 Vol.-%, oder 5 bis 15 Vol.-%, bzw. bis 10 Vol.-% H₂O enthält.

Typische Reaktionsgasausgangsgemische 1 sind z. B. solche, die enthalten:

| | |
|---|---|
| 6 bis 11 Vol.-% | Propen, |
| 6 bis 12 Vol.-% | Wasser, |
| > 0, häufig ≥ 0,5 oder ≥ 1 bis 10 Vol.-% | Propan, |
| ≥ 0 bis 5 Vol.-% | von Propen, Propan, Wasser, Sauerstoff, Cyclopropan und Stickstoff verschiedene Bestandteile, |
| soviel molekularen Sauerstoff, dass V₁ 1 bis 3 beträgt, | |

| | |
|---|---|
| | ≥ 0 und ≥ 3 mol-%, bezogen auf enthaltenes Propan, Cyclopropan, und als Restmenge bis zu 100 Vol.-% Gesamtmenge molekularen Stickstoff. |

D. h., erfindungsgemäße Reaktionsgasausgangsgemische 1 können auch enthalten:

| | |
|---|---|
| 6 bis 9 Vol.-% | Propylen, |
| 8 bis 18 Vol.-% | molekularer Sauerstoff, |
| 6 bis 30 Vol.-% | Propan und |
| 32 bis 72 Vol.-% | molekularer Stickstoff. |

Erfindungsgemäße Reaktionsgasausgangsgemische 2 können enthalten:

| | |
|---|---|
| 4,5 bis 8 Vol.-% | Acrolein, |
| 2,25 bis 9 Vol.-% | molekularer Sauerstoff, |
| 6 bis 30 Vol.-% | Propan, |
| 32 bis 72 Vol.-% | molekularer Stickstoff, |
| 5 bis 30 Vol.-% | Wasserdampf. |

Erfindungsgemäße Reaktionsgasausgangsgemische 1 können aber auch bis zu 20 Vol.-% H₂ enthalten.

D.h., Reaktionsgasgemische 1 des erfindungsgemäßen Verfahrens können auch enthalten:

| | |
|---|---|
| 4 bis 25 Vol.-% | Propylen, |
| 6 bis 70 Vol.-% | Propan, |
| 5 bis 60 Vol.-% | H₂O, |
| 8 bis 65 Vol.-% | O₂ und |
| 0,3 bis 20 Vol.-% | H₂. |

Das erfindungsgemäße Verfahren ist aber auch dann günstig, wenn das Reaktionsgasausgangsgemisch 1 0,1 bis 30 Vol.-% CO₂ enthält.

Erfindungsgemäß mögliche Reaktionsgasausgangsgemische 2 können auch enthalten:

| | |
|---|---|
| 3 bis 25 Vol.-% | Acrolein, |
| 5 bis 65 Vol.-% | molekularer Sauerstoff, |
| 6 bis 70 Vol.-% | Propan, |
| 0,3 bis 20 Vol.-% | molekularer Wasserstoff und |
| 8 bis 65 Vol.-% | Wasserdampf. |

Die Abtrennung der Acrylsäure vom Produktgasgemisch 2 kann wie in den Verfahren des Standes der Technik beschrieben erfolgen. Üblicherweise wird dazu die Acrylsäure (normalerweise begleitet von im Produktgasgemisch 2 enthaltener Nebenproduktpropionsäure) in einer ersten Trennzone aus dem Produktgasgemisch 2 in die kondensierte Phase überführt (die dabei in der Regel verbleibende Gasphase wird in dieser Schrift als Restgas bezeichnet und kann gegebenenfalls in der Partialoxidation nicht umgesetztes Propylen enthalten). Als Verfahren zur Überführung von im Produktgasgemisch 2 enthaltener Acrylsäure in die kondensierte Phase kommen in der Trennzone 1 beim erfindungsgemäßen Verfahren prinzipiell alle im Stand der Technik diesbezüglich bekannten Verfahren in Betracht. Sie sind im wesentlichen dadurch gekennzeichnet, dass man das Zielprodukt (die Acrylsäure) durch absorptive und/oder kondensative (Abkühlen) Maßnahmen aus der gasförmigen in die kondensierte Phase überführt.

Als Absorptionsmittel kommen dabei z. B. Wasser, wässrige Lösung und/oder organische (insbesondere hydrophobe) Lösungsmittel in Betracht (vgl. DE-A 103 36 386, DE-A 196 31 645, DE-A 195 01 325, EP-A 982 289, DE-A 198 38 845, WO 02/076917, EP-A 695 736, EP-A 778 225, EP-A 1 041 062, EP-A 982 287, EP-A 982 288, US 2004/0242826, EP-A 792 867, EP-A 784 046, EP-A 695 736 und in die diesen Schriften diesbezüglich zitierte Literatur).

Die im Produktgasgemisch 2 enthaltene Acrylsäure kann aber auch durch vollständige oder auch durch fraktionierende Kondensation in die kondensierte Phase überführt werden (z. B. WO 04/035514, DE-A 199 24 532, DE-A 198 14 387, DE-A 197 40 253, DE-A 197 40 252, DE-A 196 27 847 und die in diesen Schriften diesbezüglich zitierte Literatur).

Sowohl die absorptive als auch die kondensative Überführung der Acrylsäure in die flüssige Phase werden üblicherweise in trennwirksame Einbauten (zur Vergrößerung der Austauschoberfläche) enthaltenden Trennkolonnen vorgenommen. Als trennwirksame Einbauten kommen dabei alle bekannten Einbauten in Betracht. D. h., sowohl Böden wie Glockenböden, Dual-Flow-Böden, oder Ventilböden, Füllkörper wie z. B. Raschig-Ringe, oder Packungen, wie z. B. Sulzer-Packungen, können als trennwirksame Einbauten verwendet werden. In die Trennkolonne wird das Produktgasgemisch 2 dabei in der Regel von unten nach oben aufsteigend geführt. Im Rahmen einer absorptiven Kondensation wird das Absorptionsmittel in der Trennkolonne normalerweise von oben nach unten bewegt (geführt). Das flüssig ablaufende Absorbat bildet die die Acrylsäure (und schwerer sowie ähnlich wie diese siedende Nebenkomponenten wie Propionsäure) enthaltende kondensierte Phase. Bei der fraktionierenden Kondensation erfolgt die Kondensation der schwerer flüchtigen Bestandteile des Produktgasgemischs 2 in sich selbst aufsteigend. Das die Acrylsäure angereichert enthaltende Kondensat wird in der Regel über Seitenabzug aus der Kondensationskolonne herausgeführt. Selbstredend können Absorption und Kondensation auch einander überlagernd angewendet werden. Dies ist z. B. immer dann gegeben, wenn man dem System beim Absorptionsprozess durch direkte und/oder indirekte Kühlung zusätzlich Wärme entzieht.

Vorzugsweise führt man das Produktgasgemisch 2 mit einer durch indirekte Kühlung, oder durch direkte Kühlung oder durch direkte und indirekte Kühlung verringerten Temperatur in die Trennkolonne. Die indirekte Kühlung wird dabei in an sich bekannter Weise in indirekten Wärmeaustauschern vorgenommen, während zur direkten Kühlung üblicherweise in einer Quenchvorrichtung vorgekühltes Absorptionsmittel oder vorgekühlte Sumpfflüssigkeit aus der Trennkolonne in das Produktgasgemisch 2 versprüht wird. Gemeinsames Merkmal der vorbeschriebenen absorptiven und/oder kondensativen Verfahren (Trennverfahren) ist, dass am Kopf der jeweiligen trennwirksame Einbauten enthaltenden Trennkolonne, in deren unteren Teil das Produktgasgemisch 2, wie beschrieben zweckmäßig nach vorheriger direkter und/oder indirekter Kühlung desselben, üblicherweise zugeführt wird, normalerweise ein Restgasstrom verbleibt, der hauptsächlich diejenigen Bestandteile des Produktgasgemischs 2 enthält, deren Siedepunkt bei Normaldruck (1 bar) ≤ -20°C beträgt (d. h., die schwer kondensierbaren oder auch leichter flüchtigen Bestandteile).

Dazu zählen z. B. als inertes Verdünnungsgas in der Partialoxidation mitverwendeter molekularer Stickstoff, in der Partialoxidation relativ zur Reaktionsstöchiometrie verbliebener überschüssiger molekularer Sauerstoff, als Nebenprodukte gebildete oder als inerte Verdünnungsgase im Reaktionsgasausgangsgemisch 1 mitverwendete Kohlenoxide, aber auch in der Partialoxidation nicht umgesetztes Propylen und nicht umgesetztes Cyclopropan. In der Regel wird das verbliebene Restgas z. B. auch noch Anteile von Wasserdampf enthalten. Erfindungsgemäß zweckmäßig wird man wenigstens eine Teilmenge eines solchen Restgases als Bestandteil des Reaktionsgasausgangsgemischs 1 in die Partialoxidation rückführen. Eine solche Kreisgasführung kann anwendungstechnisch zweckmäßig auch über eine der erfindungsgemäßen Partialoxidation als Propylenquelle vorgeschaltete heterogen katalysierte partielle Dehydrierung und/oder Oxidehydrierung von Propan erfolgen. Häufig wird man beim erfindungsgemäßen Verfahren wenigstens 10 Vol.-%, oder wenigstens 20 Vol.-%, oder wenigstens 30 Vol.-%, meist jedoch nicht mehr als 80 Vol.-%, bzw. nicht mehr als 60 Vol.-%, bzw. nicht mehr als 40 Vol.-% des Restgases in die Partialoxidation rückführen (in der Regel jedoch möglichst vollständig, die darin enthaltene Gesamtmenge an nicht umgesetztem Propan und/oder Propen und mit diesen an nicht umgesetztem Cyclopropan). Ein Teil dieser Rückführung kann auch in die zweite Reaktionsstufe hinein erfolgen, d. h., als Bestandteil des Reaktionsgasausgangsgemischs 2.

Eine wie beschrieben durchgeführte Kreisgasführung kann einerseits als Inertgasquelle fungieren und erhöht in der Regel die angestrebte Zielproduktausbeute (bezogen auf eingesetzte Rohstoffmenge). Prinzipiell kann aber auch die Gesamtmenge und/oder eine Teilmenge des Restgases wie z. B. in der EP-A 925 272 beschrieben, der Verbrennung zugeführt werden (z. B. zur Energieerzeugung).

Absorptive und/oder kondensative Abtrennungen von Acrylsäure aus Produktgasgemischen 2 finden sich auch beschrieben in den Schriften EP-A 1 388 533, EP-A 1 388 532, DE-A 102 35 847, WO 98/01415, EP-A 1 015 411, EP-A 1 015 410, WO 99/50219, WO 00/53560, WO 02/09839, DE-A 102 35 847, WO 03/041833, DE-A 102 23 058, DE-A 102 43 625, DE-A 103 36 386, EP-A 854 129, US-A 4,317,926, DE-A 198 37 520, DE-A 196 06 877, DE-A 195 01 325, DE-A 102 47 240, DE-A 197 40 253, EP-A 695 736, EP-A 1 041 062, EP-A 117 146, DE-A 43 08 087, DE-A 43 35 172, DE-A 44 36 243, DE-A 103 32 758 sowie DE-A 199 24 533.

Eine absorptive und/oder kondensative Abtrennung der Acrylsäure aus dem Produktgasgemisch 2 kann aber auch wie in der DE-A 103 36 386, der DE-A 101 15 277, DE-A 196 06 877, EP-A 920 408, EP-A 1 068 174, der EP-A 1 066 239, der EP-A 1 066 240, der WO 00/53560, der WO 00/53561, der DE-A 100 53 086, der WO 01/96271 bzw. wie in der DE-A 10 2004 032 129 und deren äquivalenten Schutzrechten beschrieben durchgeführt werden. Günstige Abtrennweisen sind auch die in den Schriften WO 04/063138, WO 04/35514, DE-A 102 43 625 und DE-A 102 35 847 beschriebenen Verfahren. Grundsätzlich kann die Acrylsäure aus dem Produktgasgemisch 2 in der ersten Trennzone auch ausgefroren werden.

Die weitere Abtrennung der Acrylsäure aus der kondensierten Phase kann nun beim erfindungsgemäßen Verfahren je nach in der Trennzone 1 angewandter Verfahrensweise, sowie je nach den im einzelnen für die Partialoxidation gewählten und damit das sonstige Nebenkomponentenspektrum bestimmenden Verfahrensbedingungen (Reaktionstemperatur, gewählte inerte Verdünnungsgase, gewählte Katalysatoren, Gehalt an und molares Verhältnis der Reaktanden im Reaktionsgasausgangsgemisch 1 etc.) durch unterschiedlichste Kombinationen unterschiedlichster thermischer Trennverfahren bis zum gewünschten Reinheitsgrad der Acrylsäure in einer nachfolgenden Trennzone 2 vorgenommen werden. Dies können z. B. Kombinationen von extraktiven, desorptiven, kristallisativen, rektifikativen, azeotrop-destillativen, azeotrop-rektifikativen, destillativen und/oder Strippverfahren etc. sein.

Selbstverständlich werden alle in den Trennzonen 1,2 ausgeführten Verfahrensschritte polymerisationsinhibiert durchgeführt. Dabei kann wie im aufgeführten Stand der Technik beschrieben vorgegangen werden. Eine herausragende Position unter der Gesamtmenge der verfügbaren Acrylsäure-Prozessstabilisatoren nehmen Dibenzo-1,4-thiazin (PTZ), 4-Hydroxy-2,2,6,6-tetramethylpiperidin-1-oxyl (4-OH-TEMPO) und p-Methoxyphenol (MEHQ) ein. Sie können z. B. entweder jeweils für sich, oder paarweise oder als Dreiergemisch Bestandteil der erfindungsgemäß kristallisativ zu behandelnden acrylsäurehaltigen flüssigen Phase P sein. Üblicherweise beträgt die Gesamtmenge von an in der flüssigen Phase P enthaltenen Polymerisationsinhibitoren, bezogen auf darin enthaltene Acrylsäure, 0,001 bis 2 Gew.-%.

Der Vorteil des erfindungsgemäßen Verfahrens liegt darin begründet, dass es auf vergleichsweise einfache Art und Weise die Herstellung von an Propionsäure besonders armer bzw. freier Acrylsäure gestattet, ohne dass es dazu zwingend einer kristallisativen Abtrennung von Acrylsäure in der Trennzone 2 bedarf.

Abschließend sei nochmals festgehalten, dass wenigstens eine Teilmenge des bei der Überführung (in der Trennzone 1) der Acrylsäure aus dem Produktgasgemisch 2 in die kondensierte Phase verbleibenden Restgases in die erste Reaktionsstufe und/oder in die zweite Reaktionsstufe rückgeführt werden kann (als Kreisgas).

Dieses Kreisgas kann gegebenenfalls nicht umgesetztes Propylen enthalten. Erfindungsgemäß bevorzugt wird das im Reaktionsgasausgangsgemisch 1 enthaltene Propylen demselben jedoch zu wenigstens 25 mol-%, besser zu wenigstens 50 mol-%, besonders bevorzugt zu wenigstens 75 mol-%, meist zu wenigstens 85 mol-%, oder zu wenigstens 90 bzw. wenigstens 95 mol-% und teilweise sogar zu 100 mol-% als von Kreisgas verschiedenes Roh-Propylen zugesetzt.

Wird das im Reaktionsgasausgangsgemisch 1 enthaltene Propylen dem Reaktionsgasausgangsgemisch 1 wenigstens teilweise aus einer partiellen Dehydrierung (z. B. homogen und/oder heterogen katalysiert, im Beisein und/oder unter Ausschluss von molekularem Sauerstoff) zugeführt (normalerweise in Begleitung von in der partiellen Dehydrierung nicht umgesetztem Propan), kann wenigstens eine Teilmenge des bei der Überführung der Acrylsäure aus dem Produktgasgemisch 2 in die kondensierte Phase verbleibenden Restgas in die partielle Dehydrierung des Propan rückgeführt werden.

Ferner sei noch festgehalten, dass die vorliegende Erfindung Verfahren umfasst, bei denen sich an das erfindungsgemäße Verfahren zur Herstellung von Acrylsäure ein Verfahren der radikalischen Polymerisation (insbesondere zur Herstellung von Wasser superabsorbierenden Polyacrylsäuren und/oder deren teil- bzw. vollneutralisierten Alkali (vorzugsweise Na)metallsalzen) anschließt, in welchem erfindungsgemäß hergestellte Acrylsäure zur Herstellung von Polymerisaten radikalisch einpolymersiert wird.

Auch umfasst die vorliegende Erfindung Verfahren, bei denen sich an das erfindungsgemäße Verfahren zur Herstellung von Acrylsäure ein Verfahren zur Herstellung von Acrylsäureestern anschließt, in welchem erfindungsgemäß hergestellte Acrylsäure mit Alkoholen (vorzugsweise Alkanolen, besonders bevorzugt C₁- bis C₁₂-Alkanolen) (in der Regel säurekatalysiert) verestert wird.

An das Verfahren der Veresterung kann sich wiederum ein Verfahren der radikalischen Polymerisation anschließen, in welchem so hergestellter Acrylsäureester einpolymerisiert wird.

### Beispiel und Vergleichsbeispiel

### I. Zweistufige heterogen katalysierte Partialoxidation von Propylen zu Acrylsäure in Abwesenheit und im Beisein von Cyclopropan

### A) Allgemeiner Versuchsaufbau der Reaktionsvorrichtung

### Reaktor für die erste Oxidationsstufe (1. Reaktionsstufe)

Der Reaktor bestand aus einem doppelwandigen Zylinder aus Edelstahl (zylindrisches Führungsrohr, umgeben von einem zylindrischen Außenbehälter). Die Wanddicken betrugen überall 2 bis 5 mm.

Der Innendurchmesser des äußeren Zylinders betrug 91 mm. Der Innendurchmesser des Führungsrohres betrug ca. 60 mm.

Oben und unten war der doppelwandige Zylinder durch einen Deckel beziehungsweise Boden abgeschlossen.

Das Kontaktrohr (400 cm Gesamtlänge, 26 mm Innendurchmesser, 30 mm Außendurchmesser, 2 mm Wandstärke, Edelstahl) war im Führungsrohr des zylindrischen Behälters so untergebracht, dass es am oberen bzw. unteren Ende desselben (abgedichtet) durch den Deckel bzw. Boden jeweils gerade herausragte. Das Wärmeaustauschmittel (Salzschmelze, bestehend aus 53 Gew.-% Kaliumnitrat, 40 Gew.-% Natriumnitrit und 7 Gew.-% Natriumnitrat) war im zylindrischen Behälter eingeschlossen. Um über die gesamte im zylindrischen Behälter befindliche Kontaktrohrlänge (400 cm) möglichst gleichmäßige thermische Randbedingungen an der Außenwand des Kontaktrohres zu gewährleisten, wurde das Wärmeaustauschmittel mittels einer Propellerpumpe umgepumpt.

Durch eine auf den Außenmantel aufgebrachte elektrische Heizung konnte die Temperatur des Wärmeaustauschmittels auf das gewünschte Niveau geregelt werden. Im übrigen bestand Luftkühlung.

| | |
|---|---|
| Reaktorbeschickung: | Über den Erststufenreaktor betrachtet wurden die Salzschmelze und das Beschickungsgasgemisch des Erststufenreaktors im Gleichstrom geführt. Das Beschickungsgasgemisch trat unten in den Erststufenreaktor ein. Es wurde jeweils mit einer Temperatur von 165°C ins Reaktionsrohr geführt. |
| | |
| | Die Salzschmelze trat unten mit einer Temperatur T^{ein} in das zylindrische Führungsrohr ein und oben mit einer Temperatur T^{aus} aus dem zylindrischen Führungsrohr aus, die bis zu 2°C oberhalb von T^{ein} lag. |
| | |
| | T^{ein} wurde so eingestellt, dass sich am Ausgang der ersten Oxidationsstufe stets ein Propylenumsatz von 97,8 ± 0,1 mol-% bei einfachem Durchgang ergab. |
| | |
| Kontaktrohrbeschickung: (von unten nach oben) | Abschnitt A: 90 cm Länge |
| | Vorschüttung aus Steatit-Kugeln eines Durchmessers von 4-5 mm. |
| | |
| | Abschnitt B: 100 cm Länge |
| | Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt C. |
| | |
| | Abschnitt C: 200 cm Länge |
| | Katalysatorbeschickung mit ringförmigem (5 mm x 3 mm x 2 mm = Außendurchmesser x Länge x Innendurchmesser) Vollkatalysator gemäß Beispiel 1 der DE-A 100 46 957 (Stöchiometrie: |
| | [Bi₂W₂O₉x2WO₃]_{0,5} [Mo₁₂CO_{5,5}Fe_{2,94}Si_{1,59}K_{0,08}Oₓ]₁). |
| | |
| | Abschnitt D: 10 cm Länge |
| | Nachschüttung aus Steatit-Ringen der Geometrie 7 mm x 3 mm x 4 mm (Außendurchmesser x Länge x Innendurchmesser) |

Zwischenkühlung und Sauerstoffzwischeneinspeisung (reiner O₂ als Sekundärgas)

Das den ersten Festbettreaktor verlassende Produktgasgemisch 1 wurde zum Zweck der Zwischenkühlung (indirekt mittels Luft) durch ein Verbindungsrohr (40 cm Länge, 26 mm Innendurchmesser, 30 mm Außendurchmesser, 2 mm Wandstärke, Edelstahl, umwickelt mit 1 cm Isoliermaterial) geführt, das auf einer Länge von 20 cm zentriert untergebracht, mit einer Inertschüttung aus Steatit-Ringen der Geometrie 7 mm x 3 mm x 4 mm (Außendurchmesser x Länge x Innendurchmesser) beschickt und unmittelbar an das Erststufenkontaktrohr angeflanscht war.

Das Produktgasgemisch 1 trat stets mit einer Temperatur von ≥ T^{ein} (erste Stufe) in das Verbindungsrohr ein und verließ es mit einer oberhalb von 200 °C und unterhalb von 270 °C gelegenen Temperatur.

Am Ende des Verbindungsrohres wurde dem abgekühlten Produktgasgemisch 1 auf dem Druckniveau des Produktgasgemisches 1 befindlicher molekularer Sauerstoff zudosiert. Das resultierende Gasgemisch (Beschickungsgasgemisch für die zweite Oxidationsstufe) wurde unmittelbar in das Zweitstufenkontaktrohr geführt, an welchem das oben genannte Verbindungsrohr mit seinem anderen Ende ebenfalls angeflanscht war. Die Menge an zudosiertem molekularem Sauerstoff wurde so bemessen, dass das molare Verhältnis von im resultierenden Gasgemisch befindlichen O₂ zu im resultierenden Gasgemisch befindlichen Acrolein 1,3 betrug.

### Reaktor für die zweite Oxidationsstufe (2. Reaktionsstufe)

Es wurde ein Kontaktrohr-Festbettreaktor verwendet, der mit jenem für die erste Oxidationsstufe baugleich war. Salzschmelze und Beschickungsgasgemisch wurden über den Reaktor betrachtet im Gleichstrom geführt. Die Salzschmelze trat unten ein, das Beschickungsgasgemisch ebenfalls. Die Eintrittstemperatur T^{ein} der Salzschmelze wurde so eingestellt, dass sich am Ausgang der zweiten Oxidationsstufe stets ein Acroleinumsatz von 99,3 ± 0,1 mol-% bei einfachem Durchgang ergab. T^{aus} der Salzschmelze lag bis zu 2 °C oberhalb von T^{ein}.

Die Kontaktrohrbeschickung (von unten nach oben) war:

| | |
|---|---|
| Abschnitt A: | 70 cm Länge |
| | Vorschüttung aus Steatit-Ringen der Geometrie 7 mm x 3 mm x 4 mm (Außendurchmesser x Länge x Innendurchmesser). |
| | |
| Abschnitt B: | 100 cm Länge |
| | Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt C. |
| Abschnitt C: | 200 cm Länge |
| | Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ). |
| Abschnitt D: | 30 cm Länge |
| | Nachschüttung aus Steatit-Kugeln eines Durchmessers von 4-5 mm. |

B) In Abhängigkeit von der Zusammensetzung des Reaktionsgasausgangsgemischs 1 der ersten Oxidationsstufe erzielte Ergebnisse (die Propenbelastung wurde auf 150 Nl/l·h eingestellt, die Selektivität der Acrylsäurebildung (bilanziert über beide Reaktionsstufen und bezogen auf umgesetztes Propylen betrug stets ≥ 94 mol-%).
Die Zusammensetzung des Reaktionsgasausgangsgemischs 1 für die erste Oxidationsstufe enthielt im wesentlichen (bezogen auf das Gesamtvolumen des Reaktionsgasausgangsgemischs 1):

| | |
|---|---|
| 6,3 Vol.-% | Propylen, |
| 28 Vol.-% | Propan, |
| X Vol.-% | Cyclopropan, |
| 10,8 Vol.-% | O₂, |
| 5 Vol.-% | H₂O und |
| als Restmenge N₂. | |

Aus dem Produktgasgemisch 2 wurde die gebildete Acrylsäure durch Direktkühlung mit vorab gebildetem, auf 4°C abgekühltem und mit Hydrochinon polymerisationsinhibierten, Kondensat auskondensiert. Die nachfolgende Tabelle zeigt den Gewichtsanteil Y der im Kondensat enthaltenen Menge an Propionsäure, bezogen auf die darin enthaltene Menge Acrylsäure in Abhängigkeit von der im Reaktionsgasausgangsgemisch 1 enthaltenen Menge X* an Cyclopropan, hier jedoch angegeben in mol.% relativ zur im Reaktionsgasausgangsgemisch enthaltenen molaren Menge an Propylen.

**Tabelle**

| X* (mol-%) | Y (Gew.ppm) |
|---|---|
| 0,063 | 483 |
| 0,95 | 1114 |

Im Hinblick auf die obengenannten Lehren sind zahlreiche Änderungen und Abweichungen von der vorliegenden Erfindung möglich.

Man kann deshalb davon ausgehen, dass die Erfindung, im Rahmen der beigefügten Ansprüche, anders als hierin spezifisch beschrieben, ausgeführt werden kann.

## Patentansprüche

1. Verfahren der heterogen katalysierten partiellen Gasphasenoxidation von Propylen zu Acrylsäure, bei dem man in einer ersten Reaktionszone ein Propylen und molekularen Sauerstoff als Reaktanden sowie wenigstens Propan als inertes Verdünnungsgas enthaltendes Reaktionsgasausgangsgemisch 1, das den mole-kularen Sauerstoff und das Propylen in einem molaren Verhältnis O₂ : C₃H₆ ≥ 1 enthält, zunächst in einer ersten Reaktionsstufe bei erhöhter Temperatur so durch wenigstens ein erstes Katalysatorbett führt, dessen Katalysatoren als Aktivmasse wenigstens ein Mo, Fe und Bi enthaltendes Multimetalloxid aufweisen, dass der Propylenumsatz bei einmaligem Durchgang durch das Katalysatorbett ≥ 90 mol-% und die damit einhergehende Selektivität S^{AC} der Acroleinbildung sowie der Acrylsäurenebenproduktbildung zusammen ≥ 80 mol-% betragen, die Temperatur des die erste Reaktionsstufe verlassenden Produktgasgemischs 1 durch direkte Kühlung, oder durch indirekte Kühlung, oder durch direkte und indirekte Kühlung gegebenenfalls verringert, und dem Produktgasgemisch 1 gegebenenfalls Sekundärgas in Form von molekularem Sauerstoff, oder Inertgas, oder molekularem Sauerstoff und Inertgas zugibt, und danach das Produktgasgemisch 1 als Acrolein und molekularen Sauerstoff als Reaktanden sowie wenigstens Propan als inertes Verdünnungsgas enthaltendes Reaktionsgasausgangsgemisch 2, das den molekularen Sauerstoff und das Acrolein in einem molaren Verhältnis O₂ : C₃H₄O ≥ 0,5 enthält, in einer zweiten Reaktionsstufe bei erhöhter Temperatur und unter Bildung eines Produktgasgemischs 2 so durch wenigstens ein zweites Katalysatorbett führt, dessen Katalysatoren als Aktivmasse wenigstens ein Mo und V enthaltendes Multimetalloxid aufweisen, dass der Acroleinumsatz bei einmaligem Durchgang durch das Katalysatorbett ≥ 95 mol-% und die Selektivität S^{AA} der über beide Reaktionsstufen bilanzierten Acrylsäurebildung, bezogen auf umgesetztes Propylen, ≥ 70 mol-% beträgt, **dadurch gekennzeichnet, dass** das Reaktionsgasausgangsgemisch 1, bezogen auf die darin enthaltene molare Menge an Propan, ≤ 3 mol-% Cyclopropan enthält und das für das Verfahren als Ausgangssubstanz benötigte Propylen als Bestandteil von Roh-Propylen zugesetzt wird, das zu mindestens 90 Gew.-% aus Propylen und zu mindestens 97 Gew.-% aus Propan und Propylen besteht.

2. Verfahren nach Anspruch 1, **dadurch gekennzeichnet, dass** der Acroleinumsatz bei einmaligem Durchgang durch das Katalysatorbett ≥ 96 mol-% beträgt.

3. Verfahren nach Anspruch 1, **dadurch gekennzeichnet, dass** der Acroleinumsatz bei einmaligem Durchgang durch das Katalysatorbett ≥ 97 mol-% beträgt.

4. Verfahren nach Anspruch 1, **dadurch gekennzeichnet, dass** der Acroleinumsatz bei einmaligem Durchgang durch das Katalysatorbett ≥98 mol-% beträgt.

5. Verfahren nach Anspruch 1, **dadurch gekennzeichnet, dass** der Acroleinumsatz bei einmaligem Durchgang durch das Katalysatorbett ≥ 99 mol-% beträgt.

6. Verfahren nach einem der Ansprüche 1 bis 5, **dadurch gekennzeichnet, dass** das Reaktionsgasausgangsgemisch 1, bezogen auf die darin enthaltene Menge an Propan, ≤ 2 mol-% Cyclopropan enthält.

7. Verfahren nach einem der Ansprüche 1 bis 5, **dadurch gekennzeichnet, dass** das Reaktionsgasausgangsgemisch 1, bezogen auf die darin enthaltene Menge an Propan, ≤ 1 mol-% Cyclopropan enthält.

8. Verfahren nach einem der Ansprüche 1 bis 5, **dadurch gekennzeichnet, dass** das Reaktionsgasausgangsgemisch 1, bezogen auf die darin enthaltene Menge an Propan, ≤ 0,2 mol-% Cyclopropan enthält.

9. Verfahren nach einem der Ansprüche 1 bis 5, **dadurch gekennzeichnet, dass** das Reaktionsgasausgangsgemisch 1, bezogen auf die darin enthaltene Menge an Propan, ≤ 0,15 mol-% Cyclopropan enthält.

10. Verfahren nach einem der Ansprüche 1 bis 9, **dadurch gekennzeichnet, dass** der Propylenumsatz bei einmaligem Durchgang durch das Katalysatorbett ≥ 92 mol-% beträgt.

11. Verfahren nach einem der Ansprüche 1 bis 9, **dadurch gekennzeichnet, dass** der Propylenumsatz bei einmaligem Durchgang durch das Katalysatorbett ≥ 94 mol-% beträgt.

12. Verfahren nach einem der Ansprüche 1 bis 11, **dadurch gekennzeichnet, dass** das Reaktionsgasausgangsgemisch 1, bezogen auf die darin enthaltene Menge an Propan, ≥ 10 molppb Cyclopropan enthält.

13. Verfahren nach einem der Ansprüche 1 bis 11, **dadurch gekennzeichnet, dass** das Reaktionsgasausgangsgemisch 1, bezogen auf die darin enthaltene Menge an Propan, ≥ 50 molppb Cyclopropan enthält.

14. Verfahren nach einem der Ansprüche 1 bis 11, **dadurch gekennzeichnet, dass** das Reaktionsgasausgangsgemisch 1, bezogen auf die darin enthaltene Menge an Propan, ≥ 1 molppm Cyclopropan enthält.

15. Verfahren nach einem der Ansprüche 1 bis 14, **dadurch gekennzeichnet, dass** das wenigstens eine Mo, Fe und Bi enthaltende Multimetalloxid ein solches der allgemeinen Formel IV,
Mo₁₂BiₐFe_{b}X_{c}¹X_{d}²Xₑ³X_{f}⁴Oₙ (IV),
mit
X¹ = Nickel und/oder Kobalt,
X²= Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Alumium, Titan und/oder Zirkonium,
a = 0,5 bis 5,
b = 0,01 bis 5,
c = 0 bis 10,
d = 0 bis 2,
e = 0 bis 8,
f = 0 bis 10 und
n = eine Zahl, die durch die Wertigkeit und Häufigkeit der von Sauerstoff verschiedenen Elemente in IV bestimmt wird,
ist.

16. Verfahren nach einem der Ansprüche 1 bis 15, **dadurch gekennzeichnet, dass** das wenigstens eine Mo und V enthaltende Multimetalloxid ein solches der allgemeinen Formel VII,
Mo₁₂VₐX_{b}¹X_{c}²X_{d}³Xₑ⁴X_{f}⁵X_{g}⁶Oₙ (VII),
mit
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in VII bestimmt wird,
ist.

17. Verfahren nach einem der Ansprüche 1 bis 16, **dadurch gekennzeichnet, dass** die volumenspezifische Aktivität des wenigstens einen ersten Katalysatorbetts in Strömungsrichtung des Reaktionsgasausgangsgemischs 1 über die Länge des Strömungsweges wenigstens einmal zunimmt.

18. Verfahren nach einem der Ansprüche 1 bis 17, **dadurch gekennzeichnet, dass** die volumenspezifische Aktivität des wenigstens einen zweiten Katalysatorbetts in Strömungsrichtung des Reaktionsgasausgangsgemischs 2 über die Länge des Strömungsweges wenigstens einmal zunimmt.

19. Verfahren nach einem der Ansprüche 1 bis 18, **dadurch gekennzeichnet, dass** das wenigstens eine erste Katalysatorbett ein Festbett ist und seine Propenbelastung ≥ 120 NI/l·h und ≤ 250 NI/l·h beträgt.

20. Verfahren nach einem der Ansprüche 1 bis 19, **dadurch gekennzeichnet, dass** das Reaktionsgasausgangsgemisch 1 6 bis 13 Vol.-% Propylen enthält.

21. Verfahren nach einem der Ansprüche 1 bis 20, **dadurch gekennzeichnet, dass** das Reaktionsgasausgangsgemisch 1 > 0 bis 35 Vol.-% H₂O enthält.

22. Verfahren nach einem der Ansprüche 1 bis 21, **dadurch gekennzeichnet, dass** das Reaktionsgasausgangsgemisch 1 ≥ 0,01 Vol.-% Propan enthält.

23. Verfahren nach einem der Ansprüche 1 bis 21, **dadurch gekennzeichnet, dass** das Reaktionsgasausgangsgemisch 1 ≥ 1 Vol.-% Propan enthält.

24. Verfahren nach einem der Ansprüche 1 bis 21, **dadurch gekennzeichnet, dass** das Reaktionsgasausgangsgemisch 1 ≥ 5 bis ≤ 70 Vol.-% Propan enthält.

25. Verfahren nach einem der Ansprüche 1 bis 24, **dadurch gekennzeichnet, dass** das Reaktionsgasausgangsgemisch 1 ≥ 0,01 Vol.-% CO₂ enthält.

26. Verfahren nach einem der Ansprüche 1 bis 25, **dadurch gekennzeichnet, dass** das Reaktionsgasausgangsgemisch 1 ≥ 1 Vol.-% N₂ enthält.

27. Verfahren nach einem der Ansprüche 1 bis 26, **dadurch gekennzeichnet, dass** die Acrylsäure in einer Trennzone 1 aus dem Produktgasgemisch 2 durch Überführen in die kondensierte Phase abgetrennt wird.

28. Verfahren nach Anspruch 27, **dadurch gekennzeichnet, dass** die Acrylsäure durch absorptive Maßnahmen aus dem Produktgasgemisch 2 in die kondensierte Phase überführt wird.

29. Verfahren nach Anspruch 27, **dadurch gekennzeichnet, dass** die Acrylsäure durch kondensative Maßnahmen aus dem Produktgasgemisch 2 in die kondensierte Phase überführt wird.

30. Verfahren nach Anspruch 27, **dadurch gekennzeichnet, dass** die Acrylsäure durch absorptive und kondensative Maßnahmen aus dem Produktgasgemisch 2 in die kondensierte Phase überführt wird.

31. Verfahren nach Anspruch 28 oder 30, **dadurch gekennzeichnet, dass** als Absorptionsmittel Wasser oder eine wässrige Lösung verwendet wid.

32. Verfahren nach einem der Ansprüche 27 bis 31, **dadurch gekennzeichnet, dass** die Acrylsäure in einer Trennzone 2 unter Anwendung wenigstens eines thermischen Trennverfahrens von der in der Trennzone 1 erzeugten kondensierten Phase abgetrennt wird.

33. Verfahren nach einem der Ansprüche 27 bis 32, **dadurch gekennzeichnet, dass** wenigstens eine Teilmenge des bei der Überführung der Acrylsäure aus dem Produktgasgemisch 2 in die kondensierte Phase verbleibenden Restgases in die erste Reaktionsstufe und/oder in die zweite Reaktionsstufe rückgeführt wird.

34. Verfahren nach einem der Ansprüche 1 bis 33, **dadurch gekennzeichnet, dass** das im Reaktionsgasausgangsgemisch 1 enthaltene Propylen dem Reaktionsgasausgangsgemisch 1 wenigstens teilweise aus einer partiellen Dehydrierung von Propan zugeführt wird.

35. Verfahren nach Anspruch 34, **dadurch gekennzeichnet, dass** wenigstens eine Teilmenge des bei der Überführung der Acrylsäure aus dem Produktgasgemisch 2 in die kondensierte Phase verbleibenden Restgas in die partielle Dehydrierung des Propan rückgeführt wird.

36. Verfahren nach einem der Ansprüche 1 bis 35, **dadurch gekennzeichnet, dass** sich ein Verfahren zur Herstellung von Polymerisaten anschließt, in welchem nach einem Verfahren gemäß den Ansprüchen 1 bis 35 hergestellte Acrylsäure einpolymerisiert wird.

37. Verfahren nach einem der Ansprüche 1 bis 35, **dadurch gekennzeichnet, dass** sich ein Verfahren zur Herstellung von Acrylsäureester anschließt, in welchem nach einem Verfahren gemäß den Ansprüche 1 bis 35 hergestellte Acrylsäure mit einem Alkohol verestert wird.

38. Verfahren nach Anspruch 37, **dadurch gekennzeichnet, dass** sich ein Verfahren zur Herstellung von Polymerisaten anschließt, in welchem nach einem Verfahren gemäß Anspruch 37 hergestellter Acrylsäureester einpolymerisiert wird.

## Claims

1. A process for heterogeneously catalyzed partial gas phase oxidation of propylene to acrylic acid, in which, in a first reaction zone, a starting reaction gas mixture 1 which comprises propylene and molecular oxygen as reactants and at least propane as an inert diluent gas and comprises the molecular oxygen and the propylene in a molar O₂ : C₃H₆ ratio of ≥ 1 is first, in a first reaction stage at elevated temperature, conducted through at least one first catalyst bed whose catalysts have at least one multimetal oxide comprising Mo, Fe and Bi as the active composition in such a way that the propylene conversion in single pass through the catalyst bed is ≥ 90 mol% and the accompanying selectivity S^{AC} of acrolein formation and of acrylic acid by-product formation together is ≥ 80 mol%, the temperature of the product gas mixture 1 leaving the first reaction stage is optionally reduced by direct cooling or by indirect cooling or by direct and indirect cooling, and secondary gas in the form of molecular oxygen or inert gas or molecular oxygen and inert gas is optionally added to product gas mixture 1, and then product gas mixture 1, as a starting reaction gas mixture 2 which comprises acrolein and molecular oxygen as reactants and at least propane as an inert diluent gas and comprises the molecular oxygen and the acrolein in a molar O₂ : C₃H₄O ratio of ≥ 0.5, in a second reaction stage at elevated temperature and with formation of a product gas mixture 2, is conducted through at least one second catalyst bed whose catalysts have at least one multimetal oxide comprising Mo and V as the active composition in such a way that the acrolein conversion in single pass through the catalyst bed is ≥ 95 mol% and the selectivity S^{AA} of acrylic acid formation assessed over both reaction stages, based on propylene converted, is ≥ 70 mol%, wherein starting reaction gas mixture 1, based on the molar amount of propane present therein, comprises ≤ 3 mol% of cyclopropane and the propylene required as a starting substance for the process is added as a constituent of crude propylene which consists of propylene to an extent of at least 90% by weight and of propane and propylene to an extent of at least 97% by weight.

2. The process according to claim 1, wherein the acrolein conversion in single pass through the catalyst bed is ≥ 96 mol%.

3. The process according to claim 1, wherein the acrolein conversion in single pass through the catalyst bed is ≥ 97 mol%.

4. The process according to claim 1, wherein the acrolein conversion in single pass through the catalyst bed is ≥ 98 mol%.

5. The process according to claim 1, wherein the acrolein conversion in single pass through the catalyst bed is ≥ 99 mol%.

6. The process according to any of claims 1 to 5, wherein starting reaction gas mixture 1, based on the amount of propane present therein, comprises ≤ 2 mol% of cyclopropane.

7. The process according to any of claims 1 to 5, wherein starting reaction gas mixture 1, based on the amount of propane present therein, comprises ≤ 1 mol% of cyclopropane.

8. The process according to any of claims 1 to 5, wherein starting reaction gas mixture 1, based on the amount of propane present therein, comprises ≤ 0.2 mol% of cyclopropane.

9. The process according to any of claims 1 to 5, wherein starting reaction gas mixture 1, based on the amount of propane present therein, comprises ≤ 0.15 mol% of cyclopropane.

10. The process according to any of claims 1 to 9, wherein the propylene conversion in single pass through the catalyst bed is ≥ 92 mol%.

11. The process according to any of claims 1 to 9, wherein the propylene conversion in single pass through the catalyst bed is ≥ 94 mol%.

12. The process according to any of claims 1 to 11, wherein starting reaction gas mixture 1, based on the amount of propane present therein, comprises ≥ 10 molppb of cyclopropane.

13. The process according to any of claims 1 to 11, wherein starting reaction gas mixture 1, based on the amount of propane present therein, comprises ≥ 50 molppb of cyclopropane.

14. The process according to any of claims 1 to 11, wherein starting reaction gas mixture 1, based on the amount of propane present therein, comprises ≥ 1 molppm of cyclopropane.

15. The process according to any of claims 1 to 14, wherein the at least one multimetal oxide comprising Mo, Fe and Bi is one of the general formula IV
Mo₁₂BiₐFe_{b}X_{c}¹X_{d}²Xₑ³X_{f}⁴Oₙ (IV)
where
X¹= nickel and/or cobalt,
X²= thallium, an alkali metal and/or an alkaline earth metal,
X³= zinc, phosphorus, arsenic, boron, antimony, tin, cerium, lead and/or tungsten,
X⁴= silicon, aluminum, titanium and/or zirconium,
a = from 0.5 to 5,
b = from 0.01 to 5,
c = from 0 to 10,
d = from 0 to 2,
e = from 0 to 8,
f = from 0 to 10 and
n = a number which is determined by the valency and frequency of the elements in IV other than oxygen.

16. The process according to any of claims 1 to 15, wherein the at least one multimetal oxide comprising Mo and V is one of the general formula VII
Mo₁₂VₐX_{b}¹X_{c}²X_{d}³Xₑ⁴X_{f}⁵X_{g}⁶ Oₙ (VII)
where
X¹= W, Nb, Ta, Cr and/or Ce,
X²= Cu, Ni, Co, Fe, Mn and/or Zn,
X³= Sb and/or Bi,
X⁴= one or more alkali metals,
X⁵= one or more alkaline earth metals,
X⁶= Si, Al, Ti and/or Zr,
a = from 1 to 6,
b = from 0.2 to 4,
c = from 0.5 to 18,
d = from 0 to 40,
e = from 0 to 2,
f = from 0 to 4,
g = from 0 to 40 and
n = a number which is determined by the valency and frequency of the elements in VII other than oxygen.

17. The process according to any of claims 1 to 16, wherein the volume-specific activity of the at least one first catalyst bed increases at least once over the length of the flow path in flow direction of starting reaction gas mixture 1.

18. The process according to any of claims 1 to 17, wherein the volume-specific activity of the at least one second catalyst bed increases at least once over the length of the flow path in flow direction of starting reaction gas mixture 2.

19. The process according to any of claims 1 to 18, wherein the at least one first catalyst bed is a fixed bed and its propene loading is ≥ 120 1 (STP)/1·h and ≤ 250 1 (STP)/l·h.

20. The process according to any of claims 1 to 19, wherein starting reaction gas mixture 1 comprises from 6 to 13% by volume of propylene.

21. The process according to any of claims 1 to 20, wherein starting reaction gas mixture 1 comprises from > 0 to 35% by volume of H₂O.

22. The process according to any of claims 1 to 21, wherein starting reaction gas mixture 1 comprises from ≥ 0.01% by volume of propane.

23. The process according to any of claims 1 to 21, wherein starting reaction gas mixture 1 comprises from ≥ 1% by volume of propane.

24. The process according to any of claims 1 to 21, wherein starting reaction gas mixture 1 comprises from ≥ 5 to ≤ 70% by volume of propane.

25. The process according to any of claims 1 to 24, wherein starting reaction gas mixture 1 comprises from ≥ 0.01% by volume of CO₂.

26. The process according to any of claims 1 to 25, wherein starting reaction gas mixture 1 comprises from ≥ 1% by volume of N₂.

27. The process according to any of claims 1 to 26, wherein the acrylic acid is removed in a separation zone 1 from product gas mixture 2 by conversion to the condensed phase.

28. The process according to claim 27, wherein the acrylic acid is converted from product gas mixture 2 into the condensed phase by absorptive measures.

29. The process according to claim 27, wherein the acrylic acid is converted from product gas mixture 2 into the condensed phase by condensative measures.

30. The process according to claim 27, wherein the acrylic acid is converted from product gas mixture 2 into the condensed phase by absorptive and condensative measures.

31. The process according to claim 28 or 30, wherein the absorbent used is water or an aqueous solution.

32. The process according to any of claims 27 to 31, wherein the acrylic acid is removed in a separation zone 2 using at least one thermal separation process from the condensed phase obtained in separation zone 1.

33. The process according to any of claims 27 to 32, wherein at least a portion of the residual gas remaining in the conversion of the acrylic acid from product gas mixture 2 into the condensed phase is recycled into the first reaction stage and/or into the second reaction stage.

34. The process according to any of claims 1 to 33, wherein the propylene present in starting reaction gas mixture 1 is fed to starting reaction gas mixture 1 at least partly from a partial dehydrogenation of propane.

35. The process according to claim 34, wherein at least a portion of the residual gas remaining in the conversion of the acrylic acid from product gas mixture 2 into the condensed phase is recycled into the partial dehydrogenation of propane.

36. The process according to any of claims 1 to 35, which is followed by a process for preparing polymers in which acrylic acid prepared by a process according to claims 1 to 35 is polymerized.

37. The process according to any of claims 1 to 35, which is followed by a process for preparing acrylic esters in which acrylic acid prepared by a process according to claims 1 to 35 is esterified with an alcohol.

38. The process according to claim 37, which is followed by a process for preparing polymers in which acrylic ester prepared by a process according to claim 37 is polymerized.

## Revendications

1. Procédé pour l'oxydation en phase gazeuse partielle de propylène en acide acrylique, à catalyse hétérogène, dans lequel dans une première zone de réaction on fait passer à travers au moins un premier lit de catalyseurs, dont les catalyseurs comportent en tant que masse active au moins un oxyde multimétallique contenant Mo, Fe et Bi, d'abord dans un premier stade de réaction, un mélange initial de gaz de réaction 1 contenant du propylène et de l'oxygène moléculaire en tant que réactants ainsi qu'au moins du propane en tant que gaz inerte de dilution, qui contient l'oxygène moléculaire et le propylène en un rapport molaire O₂ : C₃H₆ ≥ 1, à température élevée, de manière que le taux de conversion du propylène lors d'un seul passage à travers le lit de catalyseurs soit ≥ 90 % en moles et la sélectivité S^{AC} qui l'accompagne de la formation d'acroléine ainsi que de la formation de sous-produits d'acide acrylique soient ensemble ≥ 80 % en moles, la température du mélange de gaz produit 1 quittant le premier stade de réaction est éventuellement abaissée par refroidissement direct, ou par refroidissement indirect, ou par refroidissement direct et indirect et on ajoute au mélange de gaz produit 1 éventuellement du gaz secondaire sous forme d'oxygène moléculaire, ou un gaz inerte, ou de l'oxygène moléculaire et un gaz inerte, et ensuite, dans un deuxième stade de réaction on fait passer à travers au moins un deuxième lit de catalyseurs, dont les catalyseurs comportent en tant que masse active au moins un oxyde multimétallique contenant Mo et V, le mélange de gaz produit 1, en tant que mélange initial de gaz de réaction 2 contenant de l'acroléine et de l'oxygène moléculaire en tant que réactants ainsi qu'au moins du propane en tant que gaz inerte de dilution, qui contient l'oxygène moléculaire et l'acroléine en un rapport molaire O₂ : C₃H₄O ≥ 0,5, à température élevée et avec formation d'un mélange de gaz produit 2, de manière que le taux de conversion de l'acroléine lors d'un seul passage à travers le lit de catalyseurs soit ≥ 95 % en moles et la sélectivité S^{AA} de la formation d'acide acrylique, établie sur les deux stades de réaction, par rapport au propylène mis en réaction, soit ≥ 70 % en moles, **caractérisé en ce que** le mélange initial de gaz de réaction 1 contient, par rapport à la quantité molaire de propane contenue dans celui-ci, ≤ 3 % en moles de cyclopropane et on ajoute le propylène, en tant que composant de propylène brut, requis comme substance de départ pour le procédé, qui consiste à raison d'au moins 90 % en poids en propylène et à raison d'au moins 97 % en poids en propane et propylène.

2. Procédé selon la revendication 1, **caractérisé en ce que** le taux de conversion de l'acroléine, avec un seul passage à travers le lit de catalyseur, est ≥ 96 % en moles.

3. Procédé selon la revendication 1, **caractérisé en ce que** le taux de conversion de l'acroléine, avec un seul passage à travers le lit de catalyseur, est ≥ 97 % en moles.

4. Procédé selon la revendication 1, **caractérisé en ce que** le taux de conversion de l'acroléine, avec un seul passage à travers le lit de catalyseur, est ≥ 98 % en moles.

5. Procédé selon la revendication 1, **caractérisé en ce que** le taux de conversion de l'acroléine, avec un seul passage à travers le lit de catalyseur, est ≥ 99 % en moles.

6. Procédé selon l'une quelconque des revendications 1 à 5, **caractérisé en ce que** le mélange initial de gaz de réaction 1 contient ≤ 2 % en moles de cyclopropane, par rapport à la quantité de propane contenue dans celui-ci.

7. Procédé selon l'une quelconque des revendications 1 à 5, **caractérisé en ce que** le mélange initial de gaz de réaction 1 contient ≤ 1 % en moles de cyclopropane, par rapport à la quantité de propane contenue dans celui-ci.

8. Procédé selon l'une quelconque des revendications 1 à 5, **caractérisé en ce que** le mélange initial de gaz de réaction 1 contient ≤ 0,2 % en moles de cyclopropane, par rapport à la quantité de propane contenue dans celui-ci.

9. Procédé selon l'une quelconque des revendications 1 à 5, **caractérisé en ce que** le mélange initial de gaz de réaction 1 contient ≤ 0,15 % en moles de cyclopropane, par rapport à la quantité de propane contenue dans celui-ci.

10. Procédé selon l'une quelconque des revendications 1 à 9, **caractérisé en ce que** le taux de conversion du propylène, avec un seul passage à travers le lit de catalyseur, est ≥ 92 % en moles.

11. Procédé selon l'une quelconque des revendications 1 à 9, **caractérisé en ce que** le taux de conversion du propylène, avec un seul passage à travers le lit de catalyseur, est ≥ 94 % en moles.

12. Procédé selon l'une quelconque des revendications 1 à 11, **caractérisé en ce que** le mélange initial de gaz de réaction 1 contient ≥ 10 ppb en moles de cyclopropane, par rapport à la quantité de propane contenue dans celui-ci.

13. Procédé selon l'une quelconque des revendications 1 à 11, **caractérisé en ce que** le mélange initial de gaz de réaction 1 contient, par rapport à la quantité de propane contenue dans celui-ci, ≥ 50 ppb en moles de cyclopropane.

14. Procédé selon l'une quelconque des revendications 1 à 11, **caractérisé en ce que** le mélange initial de gaz de réaction 1 contient, par rapport à la quantité de propane contenue dans celui-ci, ≥ 1 ppb en moles de cyclopropane.

15. Procédé selon l'une quelconque des revendications 1 à 14, **caractérisé en ce que** ledit au moins un oxyde multimétallique contenant Mo, Fe et Bi est un tel oxyde de formule générale IV,
Mo₁₂BiₐFe_{b}X_{c}¹X_{d}²Xₑ³X_{f}⁴Oₙ (IV)
où
X¹ = nickel et/ou cobalt,
X² = thallium, un métal alcalin et/ou un métal alcalino-terreux,
X³ = zinc, phosphore, arsenic, bore, antimoine, étain, cérium, plomb et/ou tungstène,
X⁴ = silicium, aluminium, titane et/ou zirconium,
a = 0,5 à 5,
b = 0,01 à 5,
c = 0 à 10,
d = 0 à 2,
e = 0 à 8,
f = 0 à 10 et
n = un nombre qui est déterminé par la valence et la fréquence des éléments différents de l'oxygène dans IV.

16. Procédé selon l'une quelconque des revendications 1 à 15, **caractérisé en ce que** ledit au moins un oxyde multimétallique contenant Mo et V est un tel oxyde de formule générale VII,
MO₁₂VₐX_{b}¹X_{c}²X_{d}³Xₑ⁴X_{f}⁵X_{g}⁶Oₙ (VII)
où
X¹ = W, Nb, Ta, Cr et/ou Ce,
X² = Cu, Ni, Co, Fe, Mn et/ou Zn,
X³ = Sb et/ou Bi,
X⁴ = un ou plusieurs métaux alcalins,
X⁵ = un ou plusieurs métaux alcalino-terreux,
X⁶ = Si, Al, Ti et/ou Zr,
a = 1 à 6,
b = 0,2 à 4,
c = 0,5 à 18,
d = 0 à 40,
e = 0 à 2,
f = 0 à 4,
g = 0 à 40 et
n = un nombre qui est déterminé par la valence et la fréquence des éléments différents de l'oxygène dans VII.

17. Procédé selon l'une quelconque des revendications 1 à 16, **caractérisé en ce que** l'activité spécifique volumique dudit au moins un premier lit de catalyseurs dans le sens de l'écoulement du mélange initial de gaz de réaction 1 augmente au moins une fois sur la longueur de la voie d'écoulement.

18. Procédé selon l'une quelconque des revendications 1 à 17, **caractérisé en ce que** l'activité spécifique volumique dudit au moins un deuxième lit de catalyseur dans le sens de l'écoulement du mélange initial de gaz de réaction 2 augmente au moins une fois sur la longueur de la voie d'écoulement.

19. Procédé selon l'une quelconque des revendications 1 à 18, **caractérisé en ce qu'**au moins un premier lit de catalyseurs est un lit fixe et sa charge en propène est ≥ 120 l normaux/l.h et ≤ 250 l normaux/l.h.

20. Procédé selon l'une quelconque des revendications 1 à 19, **caractérisé en ce que** le mélange initial de gaz de réaction 1 contient de 6 à 13 % en volume de propylène.

21. Procédé selon l'une quelconque des revendications 1 à 20, **caractérisé en ce que** le mélange initial de gaz de réaction 1 contient de > 0 à 35 % en volume de H₂O.

22. Procédé selon l'une quelconque des revendications 1 à 21, **caractérisé en ce que** le mélange initial de gaz de réaction 1 contient ≥ 0,01 % en volume de propane.

23. Procédé selon l'une quelconque des revendications 1 à 21, **caractérisé en ce que** le mélange initial de gaz de réaction 1 contient ≥ 1 % en volume de propane.

24. Procédé selon l'une quelconque des revendications 1 à 21, **caractérisé en ce que** le mélange initial de gaz de réaction 1 contient ≥ 5 à ≤ 70 % en volume de propane.

25. Procédé selon l'une quelconque des revendications 1 à 24, **caractérisé en ce que** le mélange initial de gaz de réaction 1 contient ≥ 0,01 % en volume de CO₂.

26. Procédé selon l'une quelconque des revendications 1 à 25, **caractérisé en ce que** le mélange initial de gaz de réaction 1 contient ≥ 1 % en volume de N₂.

27. Procédé selon l'une quelconque des revendications 1 à 26, **caractérisé en ce que** dans une zone de séparation 1 on sépare l'acide acrylique du mélange de gaz produit 2, par transfert dans la phase condensée.

28. Procédé selon la revendication 27, **caractérisé en ce qu'**on transfère par des moyens d'absorption l'acide acrylique, du mélange de gaz produit 2, dans la phase condensée.

29. Procédé selon la revendication 27, **caractérisé en ce qu'**on transfère par des moyens de condensation l'acide acrylique, du mélange de gaz produit 2, dans la phase condensée.

30. Procédé selon la revendication 27, **caractérisé en ce que** qu'on transfère par des moyens d'absorption et de condensation l'acide acrylique, du mélange de gaz produit 2, dans la phase condensée.

31. Procédé selon la revendication 28 ou 30, **caractérisé en ce qu'**on utilise comme absorbant l'eau ou une solution aqueuse.

32. Procédé selon l'une quelconque des revendications 27 à 31, **caractérisé en ce que** dans une zone de séparation 2 on sépare l'acide acrylique de la phase condensée produite dans la zone de séparation 1, en utilisant au moins un procédé de séparation thermique.

33. Procédé selon l'une quelconque des revendications 27 à 32, **caractérisé en ce qu'**au moins une quantité partielle du gaz résiduel restant lors du transfert de l'acide acrylique, du mélange de gaz produit 2, dans la phase condensée, est renvoyée dans le premier stade de réaction et/ou dans le deuxième stade de réaction.

34. Procédé selon l'une quelconque des revendications 1 à 33, **caractérisé en ce que** le propylène contenu dans le mélange initial de gaz de réaction 1 est envoyé au moins en partie à partir d'une déshydrogénation partielle du propane, au mélange initial de gaz de réaction 1.

35. Procédé selon la revendication 34, **caractérisé en ce qu'**au moins une quantité partielle du gaz résiduel restant lors du transfert de l'acide acrylique, du mélange de gaz produit 2, dans la phase condensée, est recyclée dans la déshydrogénation partielle du propane.

36. Procédé selon l'une quelconque des revendications 1 à 35, **caractérisé en ce que** fait suite un procédé pour la production de polymérisats, dans lequel de l'acide acrylique produit conformément à un procédé selon les revendications 1 à 35 est incorporé par polymérisation.

37. Procédé selon l'une quelconque des revendications 1 à 35, **caractérisé en ce que** fait suite un procédé pour la production d'esters d'acide acrylique, dans lequel de l'acide acrylique produit conformément à un procédé selon les revendications 1 à 35 est estérifié par un alcool.

38. Procédé selon la revendication 37, **caractérisé en ce que** fait suite un procédé pour la production de polymérisats, dans lequel un ester d'acide acrylique produit conformément à un procédé selon la revendication 37 est incorporé par polymérisation.
